# EUROPEAN PATENT SPECIFICATION

(11) **EP 3 893 841 B1**
(45) Date of publication and mention of the grant of the patent: **15.04.2026**
(21) Application number: 19820750.8
(22) Date of filing: 13.12.2019
(51) Int. Cl.: A61K 47/18, A61K 47/26, A61K 9/19, A61K 39/395

(54) **ANTIBODY FORMULATIONS**
ANTIKÖRPERFORMULIERUNGEN
FORMULATIONS D'ANTICORPS

(30) Priority: 14.12.2018 EP 18212591
(43) Date of publication of application: 20.10.2021
(73) Proprietor: MorphoSys AG, 82152 Planegg (DE)
(72) Inventor: BROCKS, Bodo, 82205 Gilching (DE); KELLERER, Robert, 85283 Wolnzach (DE)
(74) Representative: Elkington and Fife LLP
(86) International application number: PCT/EP2019/085049
(87) International publication number: WO 2020/120730

(56) References cited:
- WO-A1-2018/204405
- WO-A2-2013/016648
- US-A1- 2006 088 523
- US-A1- 2017 121 417

## Description

### Field of Disclosure

The present disclosure relates to formulations of a pharmaceutically active antigen binding protein, namely a monoclonal antibody. In particular, the present disclosure relates to a stable lyophilized pharmaceutical formulation of an anti-CD38 antibody, a reconstituted liquid formulation of such lyophilized formulation, and to methods of making and using such lyophilized and reconstituted formulations.

### Background

The pharmaceutical use of antibodies has increased over the past years. In many instances such antibodies are injected via the intravenous (IV) route. Alternative administration pathways are subcutaneous or intramuscular injection, which offer potential advantages, e.g. in terms of patient compliance and ease of administration. Very often highly concentrated, stable pharmaceutical formulations are desired. Also very often, for reasons such as storage and handling, lyophilized formulations are prefererred.

Therapeutic antibodies are large and complex molecules, and therefore the formulation of such proteins poses special challenges. For a protein to remain biologically active, a formulation must preserve the conformational integrity of at least a core sequence of the protein's amino acids while at the same time protecting the proteins' multiple functional groups from degradation. Formulations of antibodies may have short shelf lives and the formulated antibodies may lose biological activity resulting from chemical and physical instabilities during storage. The three most common pathways for protein degradation are protein aggregation, deamidation and oxidation (Cleland et al., Critical Reviews in Therapeutic Drug Carrier Systems 10(4): 307-377 (1993)). In particular, aggregation can potentially lead to increased immune response in patients, leading to safety concerns and must be minimised or prevented.

Formulations with higher protein concentrations pose yet additional challenges, in particular with respect to protein stabilitiy, protein-protein interactions and viscosity. Viscosity is not only an issue regarding the biophysical and biochemical properties of the therapeutic protein, but also for the delivery and manufacturing of highly concentrated protein solutions. The higher the viscosity of the solution the longer it takes to inject such a viscous solution via syringe and needle. So the aspect of syringability is influenced by the viscosity and needs to be considered during the development of a high-concentration formulation. Certain antibodies also exhibit a tendency for undesired self-interactions, which makes them less suitable for a high concentration formulation. This self-interaction propensity may lead to oligomerization, aggregation and high viscosities at high protein concentrations. Opalescence is one parameter indicating physical instability of a formulation because of the presence of aggregates or liquid-liquid phase separation in solution and has been reported for monoclonal antibody (mAb) formulations.

To address these problems the development of suitable formulation compositions is of utmost importance. In order to avoid stability problems such as the formation of aggregates, protein or antibody formulations may be lyophilized.

CD38 is a type II membrane protein having function in receptor-mediated adhesion and signaling as well as mediating calcium mobilization via its ecto-enzymatic activity, catalyzing formation of cyclic ADP-ribose (cADPR) from NAD+ and also hydrolyzing cADPR into ADP-ribose (ADPR). CD38 mediates cytokine secretion and activation and proliferation of lymphocytes (Funaro et al, J Immunology 145:2390-6, 1990; Guse et al, Nature 398:70-3, 1999), and via its NAD glycohydrolase activity regulates extracellular NAD+ levels which have been implicated in modulating the regulatory T-cell compartment (Adriouch et al., 14: 1284-92, 2012; Chiarugi et al., Nature Reviews 12:741-52, 2012).

CD38 is expressed on malignant plasma cells, and other lymphocytes, and therefore, represents a therapeutic target in the treatment of multiple myeloma and other gammopathies. CD38 is also a target that is utilizied or investigated for other indications, including but not limited to solid cancers, autoimmune disorders and numerous other diseases.

Several antibodies (including bispecific antibodies and other constructs) specific for CD38 are in use or in development, including but not limited to: WO199962526 (Mayo Foundation); WO200206347 (Crucell Holland), WO2005103083 (MorphoSys AG), WO2006125640 (MorphoSys AG), WO2007042309 (MorphoSys); WO2006099875 (Genmab), WO2011154453A1 (Genmab), WO2008047242 (Sanofi-Aventis), WO2015066450 (Sanofi), WO2012092616A1 (Takeda), WO2012092612A1 (Takeda), WO2013059885 (Teva), WO2014178820 (Teva), WO2015149077 (Xencor), WO2017081211A2 (University Hamburg-Eppendorf) and WO2017091656 (Amgen, Xencor).

The present disclosure relates to the development and use of a highly stable formulation for anti-CD38 antibodies suitable for administration to patients, such as the antibody known as MOR202.

Pharmaceutical formulations for therapeutic antibodies are known in the prior art. For example, WO2013016648 discloses a pharmaceutical formulation comprising an anti-PCSK9 antibody, a histidine buffer, a non-ionic surfactant and a stabilizer. However, said document does not disclose an anti-CD38 antibody. WO2018204405 discloses a formulation comprising an anti-TIGIT antibody, a histidine buffer, polysorbate-80 as non-ionic surfactant, a non-reducing sugar and an anti-oxidant, but also does not disclose an anti-CD38 antibody. WO2017079150 discloses subcutaneous formulations comprising an anti-CD38 antibody, hyalorunidase, a histidine buffer, methionine and a non-ionic surfactant. This document teaches the addition of hyaluronidase to the composition and does not disclose MOR202 or a freeze-dried, lyophilized composition. US 2017121417 A1 describes subcutaneous formulations of anti-CD38 antibodies and their uses, in particular a pharmaceutical composition comprising an anti-CD38 antibody and a hyaluronidase. US 2006088523 A1 describes antibody formulations, including monoclonal antibodies formulated in histidine-acetate buffer, as well as a formulation comprising an antibody that binds to domain II of HER2 (for example, Pertuzumab), and a formulation comprising an antibody that binds to DR5 (for example, Apomab).

Thus, there is still need for a stable lyophilized antibody formulation for the preparation of an anti-CD38 medicament, in particular comprising the antibody MOR202.

### Summary of the Disclosure

The invention is defined by the claims.

It is an object of the present disclosure to provide formulations of anti-CD38 antibodies, in particular formulations of anti-CD38 antibodies having a suitable shelf life.

Suitable formulations for therapeutic antibodies can be aqueous pharmaceutical compositions or lyophilisates, which can be reconstituted to provide a solution for administration to a patient.

One aspect of the claimed invention, as defined in claim 1, provides a lyophilized pharmaceutical formulation comprising an anti-CD38 antibody, wherein the anti-CD38 antibody comprises a variable heavy chain of SEQ ID NO:7 and a variable light chain of SEQ ID NO:8. In another aspect of the present disclosure, the pharmaceutical formulation comprises the antibody known as MOR202.

The lyophilised pharmaceutical formulation of the invention comprising the anti-CD38 antibody, such as MOR202, comprises a non-ionic surfactant and a histidine buffer of a pH between 5.5 and 6.5.

The lyophilized pharmaceutical formulation of the invention comprises a non-ionic surfactant. The non-ionic surfactant may be polysorbate. Said polysorbate may be polysorbate 20 (PS20) or polysorbate 80 (PS80).

In some embodiments, the lyophilized pharmaceutical formulation comprises the non-ionic surfactant at a concentration of 0.05% (w/w) to 0.2% (w/w). In other embodiments, said non-ionic surfactant is at a concentration of about 0.1% (w/w) to 0.2% (w/w).

The lyophilized pharmaceutical formulation of the invention comprises a histidine buffer of a pH between 5.5 and 6.5. Said histidine buffer may be a histidine buffer of a pH of between 5.9 and 6.1. In certain aspects, said histidine buffer has a pH of about 6.0.

In one aspect, the pharmaceutical formulation comprises a histidine buffer at a concentration of 5 mM to 15 mM. In other aspects, said histidine buffer is at a concentration of 8 mM to 12 mM. In other aspects, said histidine buffer is at a concentration of about 10 mM. In other aspects, said histidine buffer is at a concentration of 10 mM.

In one aspect, the lyophillized pharmaceutical formulation comprises a stabilizer. In certain aspects, said stabilizer is a disaccharide. In other aspects, said disaccharide is sucrose. In other aspects said sucrose is at a concentration of 150 mM to 350 mM. In other aspects, said sucrose is at a concentration of 175 mM to 260 mM. In other aspects, said sucrose is at a concentration of about 260 mM. In other aspects, said sucrose is at a concentration of 260 mM.

The present disclosure also describes the lyophilized pharmaceutical formulation for said anti-CD38 antibodies, such as MOR202, prepared by the lyophilization of a liquid pharmaceutical formulation.

A further aspect of the invention, as recited in claim 6, provides a method for preparing a liquid formulation of an anti-CD38 antibody, said method comprising providing the lyophilized pharmaceutical formulation described above and reconstituting said lyophilized formulation via the addition of water.

In a further aspect, as recited in claim 7, the present invention provides a reconstituted pharmaceutical formulation of said anti-CD38 antibodies, such as MOR202, obtained by the method of the immediately preceding paragraph.

In one aspect, the present disclosure provides the reconstituted pharmaceutical formulation of said anti-CD38 antibodies, such as MOR202, wherein said anti-CD38 antibody is at a concentration of at least 55 mg/ml.

In one aspect, the present disclosure provides the reconstituted pharmaceutical formulation of said anti-CD38 antibodies, such as MOR202, wherein said anti-CD38 antibody is at a concentration of 55-75 mg/ml.

In other aspects, said anti-CD38 antibody is at a concentration of 62.5-67.5 mg/ml.

In yet other aspects, said anti-CD38 antibody is at a concentration of about 65 mg/ml. In yet other aspects, said anti-CD38 antibody is at a concentration of 65 mg/ml.

In one aspect, the reconsititued pharmaceutical formulation comprises the non-ionic surfactant at a concentration of 0.05% (w/w) to 0.2% (w/w). In other aspects, said non-ionic surfactant is at a concentration of about 0.1% (w/w).

In one aspect, the present disclosure provides a pharmaceutical composition as described above for use in a method of treating a disease or disorder in a subject, as reited in claim 12..

In certain aspects, said disease or disorder is cancer or an inflammatory disorder. In certain aspects, said cancer is a hematological cancer or a solid cancer. In certain aspects, said hematological cancer is selected from multiple myeloma, lymphoma, leukemia, or any specific type or sub-type thereof. In certain aspects, said said cancer is multiple myeloma. In certain aspects, said disease or disorder is cancer or an inflammatory disorder.

### Brief description of the drawings

**Figure 1****:** Overview of the key pre-formulations tested in Example 2.
**Figure 2****:** Test schedule during pre-formulation development.
**Figure 3****:** Results of the stability testing in the pre-formulation development.
   Figures 3A - 3B: time point 0
   Figures 3C - 3D: time point 2 weeks, 40°C / 75% RH
   Figure 3E: time point 4 weeks, 40°C / 75% RH
   Figure 3F: time point 2 weeks, 5°C
   Figure 3G: time point 4 weeks, 5°C
**Figure 4****:** Results of various test of the lead buffer system plus various concentrations of Polysorbate 20.
   Figures 4A - 4C: Results of the shear tests at T0, T24h and T48h
   Figures 4D - 4E: Results of the freeze/thaw tests
   Figure 4F: Results of the saline dilution tests
**Figure 5****:** Vials with cakes after initial lyophilization experiments.

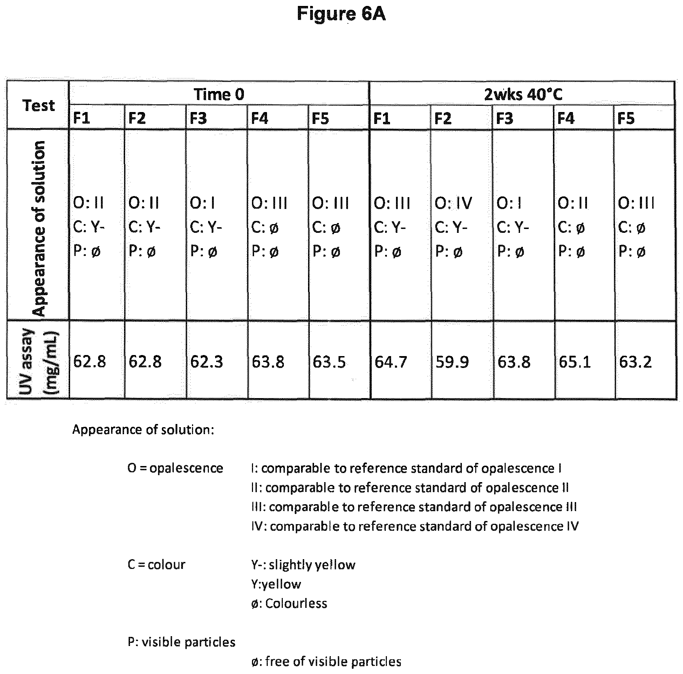

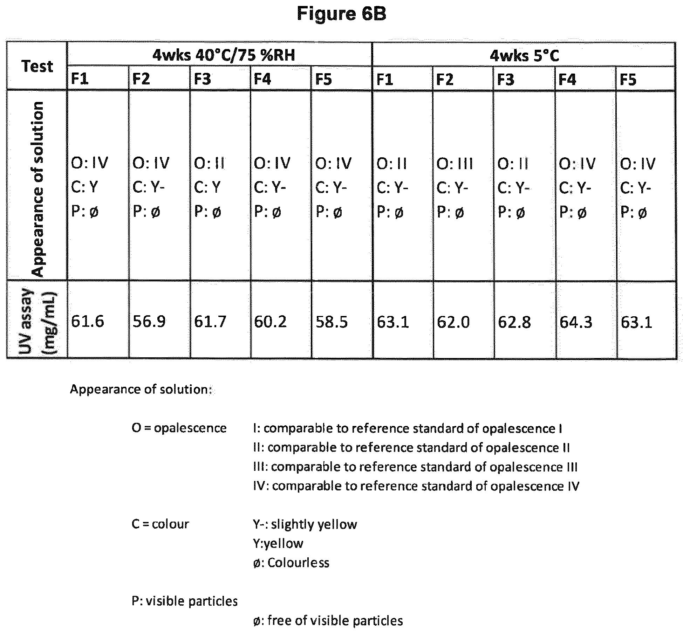

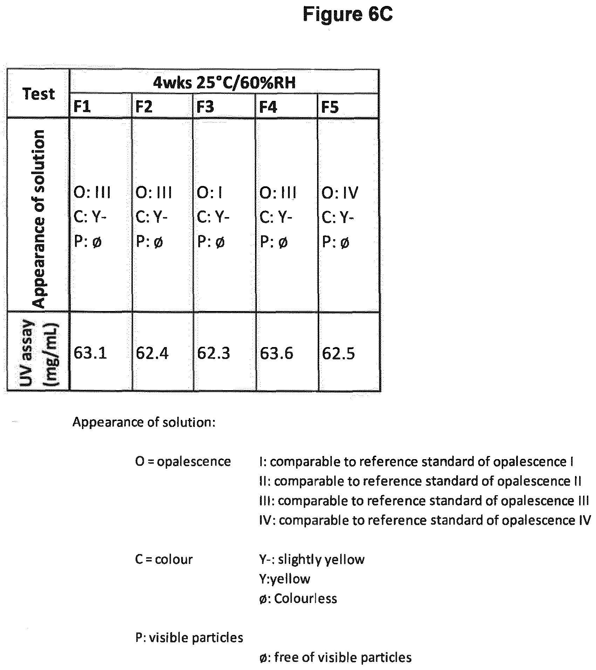

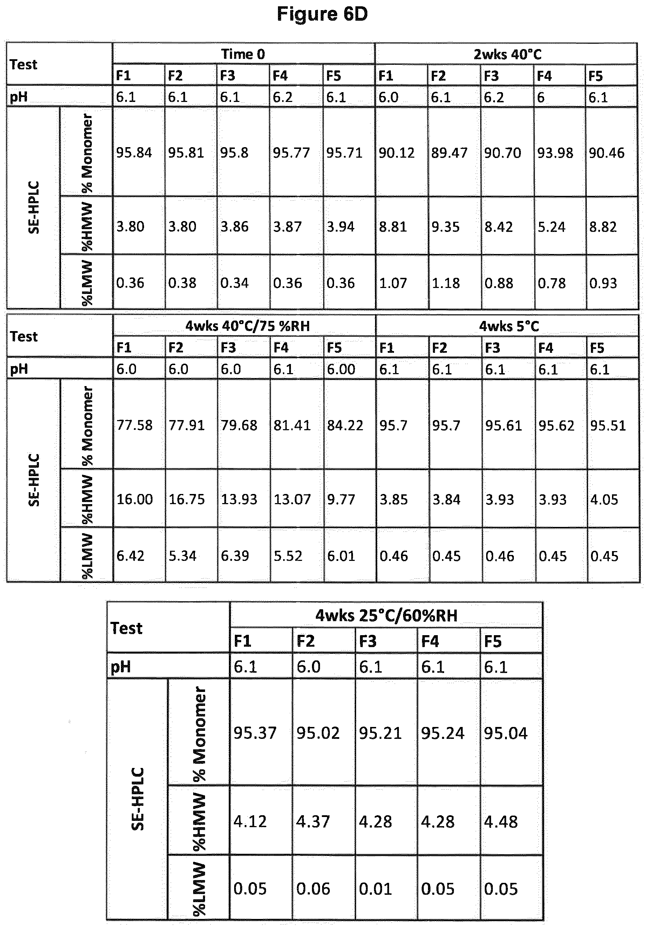

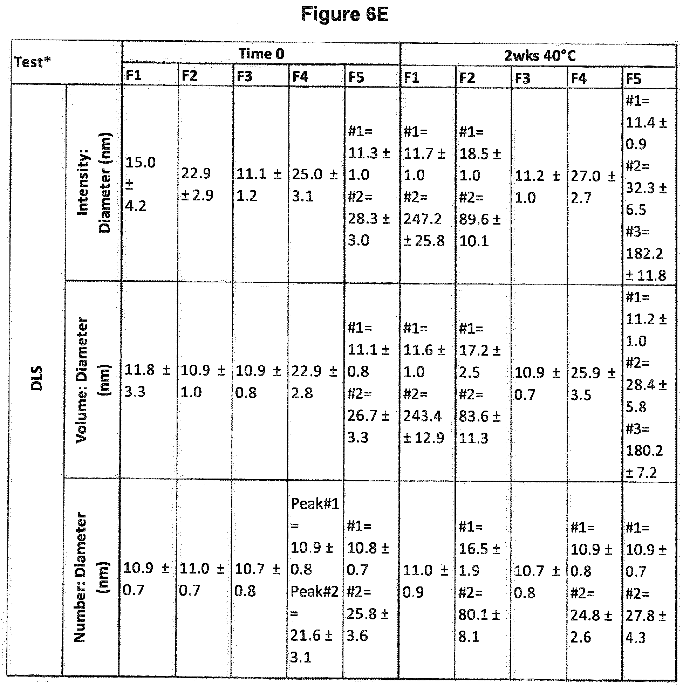

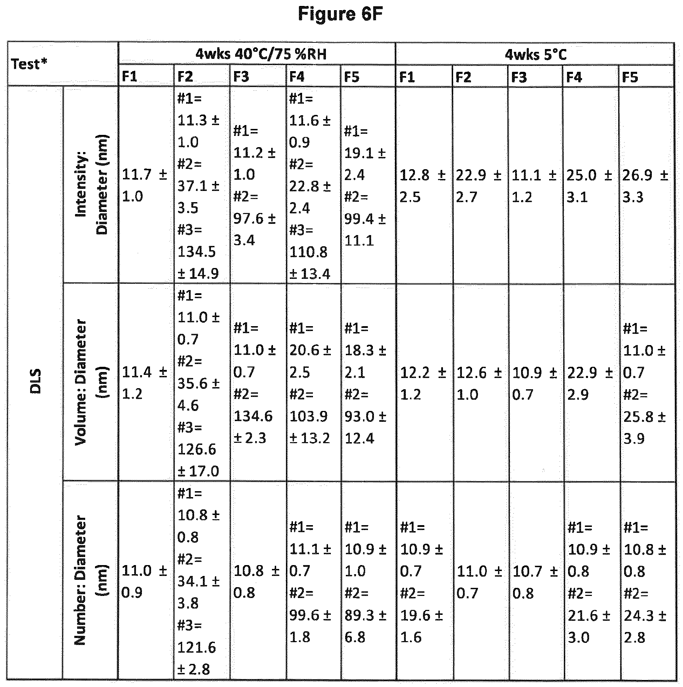

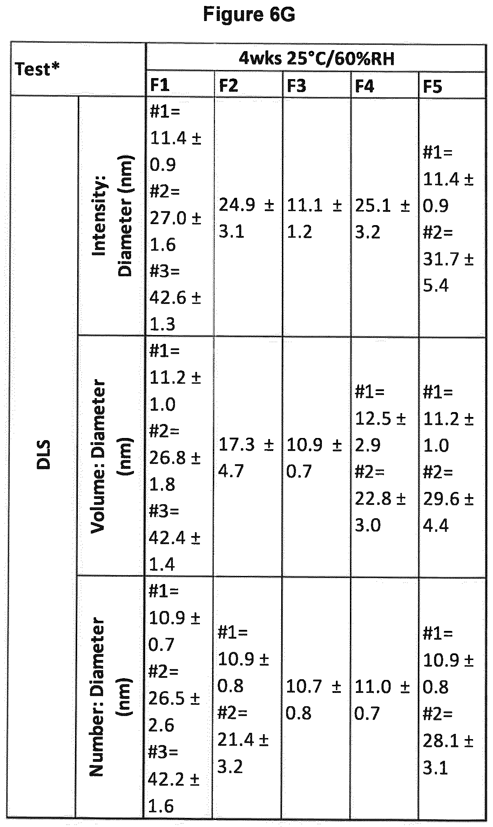

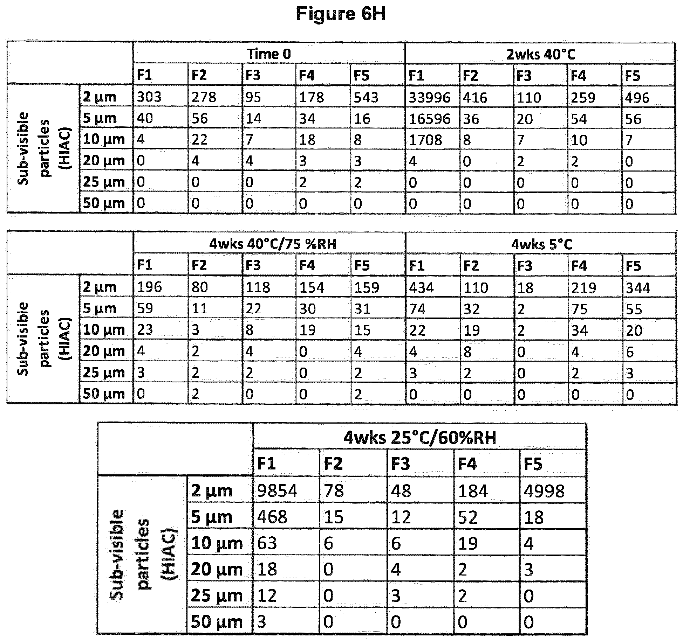

**Figure 6****:** Stability results and subvisible particles for liquid formulations.
   Figures 6A - 6C: Appearance of solutions and UV assays
   Figure 6D: pH and SE-HPLC
   Figures 6E - 6G: DLS
   Figure 6H: HIAC
**Figure 7****:** Stability results and subvisible particles for lyophilized formulations.
   Figure 7A: Appearance, UV assays and pH
   Figures 7B - 7D: DLS, appearance of cake, reconstitution time and moisture content
   Figure 7E: SE-HPLC and subvisible particles
**Figure 8****:** Analytical results of the drug product after the fifth lyophilization optimization.
**Figure 9****:** Vials with cakes after the fifth lyophilization optimization.

### Definitions and Detailed Description

The invention is defined by the claims.

It is to be understood that this disclosure is not limited to particular methods, reagents, compounds, compositions, or biological systems, which can, of course, vary. It is also to be understood that the terminology used herein is for the purpose of describing particular embodiments only, and is not intended to be limiting.

As used in this specification and the appended claims, the singular forms "a", "an", and "the" include plural referents unless the content clearly dictates otherwise. Thus, for example, reference to "a polypeptide" includes a combination of two or more polypeptides, and the like.

"About" as used herein when referring to a measurable value such as an amount, a temporal duration, and the like, is meant to encompass variations of ±20% or ±10%, including ±5%, ±1%, and ±0.1% from the specified value, as such variations are appropriate to perform the disclosed methods. Unless defined otherwise, all technical and scientific terms used herein have the same meaning as commonly understood by one of ordinary skill in the art to which the disclosure pertains. Although any methods and materials similar or equivalent to those described herein can be used in the practice for testing of the present disclosure, the preferred materials and methods are described herein, in describing and claiming the present disclosure, the following terminology will be used.

The terms "pharmaceutical formulation" or "formulation" refer to a preparation which is in such form as to permit the biological activity of the active ingredient to be effective, and which contains no additional components which are unacceptably toxic to a subject to which the formulation would be administered. Such formulations are sterile. A "sterile" formulation is aseptic or free from all living microorganisms and their spores.

The term "viscosity" refers to the internal resistance to flow exhibited by a fluid at a specified temperature; the ratio of shearing stress to rate of shear. A liquid has a viscosity of one poise if a force of 1 dyne/square centimeter causes two parallel liquid surfaces one square centimeter in area and one square centimeter apart to move past one another at a velocity of 1 cm/second. One poise equals one hundred centipoise. In one embodiment, the viscosity of the formulation comprising buffering agent and stabilizer is less than about 50 cP, less than about 45 cP, less than about 40 cP, less than about 35 cP, less than about 30 cP, less than about 25 cP, less than about 20 cP, less than about 15 cP, or less than about 10 cP.

When referring to apparent viscosity, it is understood that the value of viscosity is dependent on the conditions under which the measurement was taken, such as temperature, the rate of shear and the shear stress employed. The apparent viscosity is defined as the ratio of the shear stress to the rate of shear applied. There are a number of alternative methods for measuring apparent viscosity. For example, viscosity can be tested by a suitable cone and plate, parallel plate or other type of viscometer or rheometer.

As used herein, "buffer" refers to a buffered solution that resists changes in pH by the action of its acid-base conjugate components. The buffer of this invention preferably has a pH in the range of between 5.9 and 6.1, preferably a pH of about 6.0, and more preferably a pH of 6.0. A "histidine buffer" is a buffer comprising the amino acid histidine. Histidine buffers are preferred buffers of the present disclosure. Examples of histidine buffers include histidine hydrochloride, histidine acetate, histidine phosphate, and histidine sulfate.

As used herein, a "surfactant" refers to a surface-active agent. Examples of surfactants include polysorbate (for example, polysorbate 20 and polysorbate 80), poloxamer (for example, poloxamer 188), Triton, sodium dodecyl sulfate (SDS), sodium laurel sulfate, sodium octyl glycoside, lauryl-, myristyl-, linoleyl-, or stearyl-sulfobetaine, lauryl-, myristyl-, linoleyl- or stearyl-sarcosine, linoleyl-, myristyl-, or cetyl-betaine, lauroamidopropyl-, cocamidopropyl-, linoleamidopropyl-, myristamidopropyl-, palmidopropyl-, or isostearamidopropyl-betaine (for example lauroamidopropyl,; myristamidopropyl-, palmidopropyl-, or isostearamidopropyl- dimethylamine,; sodium methyl cocoyl-, or disodium methyl oleyl-taurate, and the MONAQUAT^{™} series (Mona Industries, Inc., Paterson, N.J.), polyethyl glycol, polypropyl glycol, and copolymers of ethylene and propylene glycol (for example Pluronics or PF68). Preferably, the surfactant is a nonionic surfactant. More preferably said nonionic surfactant is polysorbate 20. Polysorbat 20 is also know as PS20 or Tween-20. Polysorbat 80 is also know as PS80 or Tween-80.

Said surfactants may be used in different concentrations. Preferably, said surfactant is at a concentration of 0.05% (w/w) to 0.2% (w/w). More preferably, said surfactant is at a concentration of about 0.1% (w/w). More preferably, said surfactant is at a concentration of 0.1% (w/w). Preferably, said surfactant is polysorbate 20 at a concentration of 0.05% (w/w) to 0.2% (w/w). More preferably, said surfactant is polysorbate 20 at a concentration of about 0.1% (w/w). More preferably, said surfactant is polysorbate 20 at a concentration of 0.1% (w/w).

In certain embodiments, the pharmaceutical formulation according to the present disclosure may comprise a stabilizer as further excipient. Stabilizers, include, but are not limited to human serum albumin, bovine serum albumin, α-casein, globulins, α-lactalbumin, LDH, lysozyme, myoglobin, ovalbumin, and RNase A. Stabilizers also include amino acids and their metabolites, such as, glycine, alanine, arginine, betaine, leucine, lysine, glutamic acid, aspartic acid, proline, 4-hydroxyproline, sarcosine, γ-aminobutyric acid (GABA), opines (alanopine, octopine, strombine), and trimethylamine N-oxide (TMAO). Stabilizers may also include sugar alcohols and/or mono-, di- or polysaccharides, such as mannitol, sorbitol, trehalose, dextrose, lactose, sucrose, and the like. Preferably, the stabilizer is sucrose.

The term "reconstituted" as used herein in connection with the pharmaceutical formulations of the present disclosure denotes a formulation which is lyophilized and re-dissolved by addition of reconstitution medium. The reconstitution medium comprise but is not limited to water, in particular water for injection (WFI), bacteriostatic water for injection (BWFI), sodium chloride solutions (e.g. 0.9% (w/v) NaCl), glucose solutions (e.g. 5% glucose), surfactant, containing solutions (e.g. 0.01% polysorbate 20), a pH-buffered solution (e.g. phosphate-buffered solutions).

In one aspect the pharmaceutical formulation of the present disclosure is stable upon freezing and thawing, A "stable" formulation is one in which ail the protein therein essentially retain their physical stability and/or chemical stability and/or biological activity upon storage at the intended storage temperature, e.g. 2-8°C. It is desired that the formulation essentially retains its physical and chemical stability, as well as its biological activity upon storage. The storage period is generally selected based on the intended shelf-life of the formulation. Furthermore, the formulation should be stable following freezing (to, e.g., -70°C) and thawing of the formulation, for example following 1, 2 or 3 cycles of freezing and thawing. Various analytical techniques for measuring protein stability are available in the art and are reviewed in Peptide and Protein Drug Delivery', 247-301, Vincent Lee Ed., Marcel Dekker, Inc., New York, N.Y., Pubs. (1991) and Jones, A. Adv. Drug Delivery Rev. 10: 29-90 (1993), for example. Stability can be measured at a selected temperature for a selected timeperiod. Stability can be evaluated qualitatively and/or quantitatively in a variety of different ways, including evaluation of aggregate formation (for example using size exclusion chromatography, by measuring turbidity, and/or by visual inspection); by assessing charge heterogeneity using cation exchange chromatography or capillary zone electrophoresis; amino-terminal or earboxy-terminal sequence analysis; mass spectrometric analysis; SDS-PAGE analysis to compare reduced and intact antibody; peptide map (for example tryptic or LYS-C) analysis; evaluating biological activity or antigen binding function of the antibody.

In one embodiment, the pharmaceutical formulation of the present disclosure is suitable for intravenous, subcutaneous or intramuscular administration. In other embodiments, the pharmaceutical formulation of the present disclosure is administered subcutaneously. In another embodiment, the pharmaceutical formulation of the present disclosure is administered intravenously.

The term "CD38" refers to the protein known as CD38, having the following synonyms: ADP-ribosyl cyclase 1, cADPr hydrolase 1, Cyclic ADP-ribose hydrolase 1, T10.

Human CD38 (UniProt: P28907) has the amino acid sequence of:

MANCEFSPVSGDKPCCRLSRRAQLCLGVSILVLILVVVLAVVVPRWRQQWSGPGTTKRFPETVLA
RCVKYTEIHPEMRHVDCQSVWDAFKGAFISKHPCNITEEDYQPLMKLGTQTVPCNKILLWSRIKDL
AHQFTQVQRDMFTLEDTLLGYLADDLTWCGEFNTSKINYQSCPDWRKDCSNNPVSVFWKTVSRR
FAEAACDVVHVMLNGSRSKIFDKNSTFGSVEVHNLQPEKVQTLEAWVIHGGREDSRDLCQDPTIK
ELESIISKRNIQFSCKNIYRPDKFLQCVKNPEDSSCTSEI (SEQ ID NO: 9).

The term "antibody" as used herein refers to a protein comprising at least two heavy (H) chains and two light (L) chains inter-connected by disulfide bonds, which interacts with an antigen. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FR's arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. The term "antibody" includes for example, monoclonal antibodies, human antibodies, humanized antibodies, camelised antibodies and chimeric antibodies. The antibodies can be of any isotype (e.g., IgG, IgE, IgM, IgD, IgA and IgY), class (e.g., IgG1, IgG2, IgG3, IgG4, IgA1 and IgA2) or subclass. Both the light and heavy chains are divided into regions of structural and functional homology.

The phrase "antibody fragment", as used herein, refers to one or more portions of an antibody that retain the ability to specifically interact with (e.g., by binding, steric hindrance, stabilizing spatial distribution) an antigen. Examples of binding fragments include, but are not limited to, a Fab fragment, a monovalent fragment consisting of the VL, VH, CL and CH1 domains; a F(ab)2 fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., (1989) Nature 341:544-546), which consists of a VH domain; and an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al., (1988) Science 242:423-426; and Huston et al., (1988) Proc. Natl. Acad. Sci. 85:5879-5883). Such single chain antibodies are also intended to be encompassed within the term "antibody fragment". These antibody fragments are obtained using conventional techniques known to those of skill in the art, and the fragments are screened for utility in the same manner as are intact antibodies. Antibody fragments can also be incorporated into single domain antibodies, maxibodies, minibodies, intrabodies, diabodies, triabodies, tetrabodies, v-NAR and bis-scFv (see, e.g., Hollinger and Hudson, (2005) Nature Biotechnology 23:1126-1136). Antibody fragments can be grafted into scaffolds based on polypeptides such as Fibronectin type III (Fn3) (see U.S. Pat. No. 6,703,199, which describes fibronectin polypeptide monobodies). Antibody fragments can be incorporated into single chain molecules comprising a pair of tandem Fv segments (VH-CH1-VH-CH1) which, together with complementary light chain polypeptides, form a pair of antigen-binding sites (Zapata et al., (1995) Protein Eng. 8:1057-1062; and U.S. Pat. No. 5,641,870).

A "human antibody" or "human antibody fragment", as used herein, includes antibodies and antibody fragments having variable regions in which both the framework and CDR regions are derived from sequences of human origin. Human antibodies can also be isolated from synthetic libraries or from transgenic mice (e.g. xenomouse) provided the respective system yield in antibodies having variable regions in which both the framework and CDR regions are equivalent to the sequences of human origin.

Furthermore, if the antibody contains a constant region, the constant region also is derived from such sequences. Human origin includes, e.g., human germline sequences, or mutated versions of human germline sequences or antibody containing consensus framework sequences derived from human framework sequences analysis, for example, as described in Knappik et al., (2000) J Mol Biol 296:57-86).

The structures and locations of immunoglobulin variable domains, e.g., CDRs, may be defined using well known numbering schemes, e.g., the Kabat numbering scheme, the Chothia numbering scheme, or a combination of Kabat and Chothia (see, e.g., Sequences of Proteins of Immunological Interest, U.S. Department of Health and Human Services (1991), eds. Kabat et al.; Lazikani et al., (1997) J. Mol. Bio. 273:927-948); Kabat et al., (1991) Sequences of Proteins of Immunological Interest, 5th edit., NIH Publication no. 91-3242 U.S. Department of Health and Human Services; Chothia et al., (1987) J. Mol. Biol. 196:901-917; Chothia et al., (1989) Nature 342:877-883; and Al-Lazikani et al., (1997) J. Mol. Biol. 273:927-948.

A "humanized antibody" or "humanized antibody fragment" is defined herein as an antibody molecule, which has constant antibody regions derived from sequences of human origin and the variable antibody regions or parts thereof or only the CDRs are derived from another species. For example, a humanized antibody can be CDR-grafted, wherein the CDRs of the variable domain are from a non-human origin, while one or more frameworks of the variable domain are of human origin and the constant domain (if any) is of human origin.

The term "chimeric antibody" or "chimeric antibody fragment" is defined herein as an antibody molecule, which has constant antibody regions derived from, or corresponding to, sequences found in one species and variable antibody regions derived from another species. Preferably, the constant antibody regions are derived from, or corresponding to, sequences found in humans, and the variable antibody regions (e.g. VH, VL, CDR or FR regions) are derived from sequences found in a non-human animal, e.g. a mouse, rat, rabbit or hamster.

The term "isolated antibody" refers to an antibody or antibody fragment that is substantially free of other antibodies or antibody fragments having different antigenic specificities. Moreover, an isolated antibody or antibody fragment may be substantially free of other cellular material and/or chemicals. Thus, in some aspects, antibodies provided are isolated antibodies, which have been separated from antibodies with a different specificity. An isolated antibody may be a monoclonal antibody. An isolated antibody may be a recombinant monoclonal antibody. An isolated antibody that specifically binds to an epitope, isoform or variant of a target may, however, have cross-reactivity to other related antigens, e.g., from other species (e.g., species homologs).

The term "recombinant antibody", as used herein, includes all antibodies that are prepared, expressed, created or segregated by means not existing in nature. For example, antibodies isolated from a host cell transformed to express the antibody, antibodies selected and isolated from a recombinant, combinatorial human antibody library, and antibodies prepared, expressed, created or isolated by any other means that involve splicing of all or a portion of a human immunoglobulin gene, sequences to other DNA sequences or antibodies isolated from an animal (e.g., a mouse) that is transgenic or transchromosomal for human immunoglobulin genes or a hybridoma prepared therefrom. Preferably, such recombinant antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo. A recombinant antibody may be a monoclonal antibody. In an embodiment, the antibodies and antibody fragment disclosed herein are isolated from the HuCAL library (Rothe et al, J.Mol.Biol. (2008) 376, 1182-1200).

The term "monoclonal antibody" as used herein refers to a preparation of antibody molecules of single molecular composition. A monoclonal antibody composition displays a unique binding site having a unique binding specificity and affinity for particular epitopes.

In one embodiment, the antigen binding protein of the present disclosure is a monoclonal antibody or fragment thereof. In another embodiment, the antigen binding protein of the present disclosure is a monoclonal antibody. In another embodiment, the monoclonal antibody is a mouse, a chimeric, a humanized, or a human antibody. In another embodiment, the monoclonal antibody is a human antibody. In another embodiment, the monoclonal antibody is a humanized antibody. In another embodiment, the monoclonal antibody is a chimeric antibody. In another embodiment, the monoclonal antibody is a mouse antibody.

In one embodiment, the antigen binding protein of the present disclosure binds to CD38. In another embodiment, the antigen binding protein of the present disclosure binds specifically to CD38. In another embodiment, the antigen binding protein of the present disclosure is specific for CD38. In another embodiment, the antigen binding protein of the present disclosure specifically recognizes CD38.

For the avoidance of doubt, the formulations according to the claimed invention comprise an anti-CD38 antibody comprising a variable heavy chain of SEQ ID NO:7 and a variable light chain of SEQ ID NO:8.

"Percent identity" between a query amino acid sequence and a subject amino acid sequence is the "Identities" value, expressed as a percentage, that is calculated by the BLASTP algorithm when a subject amino acid sequence has 100% query coverage with a query amino acid sequence after a pair-wise BLASTP alignment is performed. Such pair-wise BLASTP alignments between a query amino acid sequence and a subject amino acid sequence are performed by using the default settings of the BLASTP algorithm available on the National Center for Biotechnology Institute's website with the filter for low complexity regions turned off. Importantly, a query amino acid sequence may be described by an amino acid sequence identified in one or more claims herein.

The query sequence may be 100% identical to the subject sequence, or it may include up to a certain integer number of amino acid alterations as compared to the subject sequence such that the % identity is less than 100%. For example, the query sequence is at least 50, 60, 70, 75, 80, 85, 90, 95, 96, 97, 98, or 99% identical to the subject sequence. Such alterations include at least one of the following: amino acid deletion, substitution (including conservative and non-conservative substitution), or insertion, and wherein said alterations may occur at the amino- or carboxy-terminal positions of the query sequence or anywhere between those terminal positions, interspersed either individually among the amino acids in the query sequence or in one or more contiguous groups within the query sequence.

"Administered" or "administration" includes but is not limited to delivery by an injectable form, such as, for example, an intravenous, intramuscular, intradermal or subcutaneous route or mucosal route, for example, as a nasal spray or aerosol for inhalation or as an ingestable solution, capsule or tablet.

A "therapeutically effective amount" of a compound or combination refers to an amount sufficient to at least partially arrest the clinical manifestations of a given disease or disorder and its complications. The amount that is effective for a particular therapeutic purpose will depend on the severity of the disease or injury as well as on the weight and general state of the subject. It will be understood that determination of an appropriate dosage may be achieved, using routine experimentation, by constructing a matrix of values and testing different points in the matrix, all of which is within the ordinary skills of a trained physician or clinical scientist.

"MOR202" is an anti-CD38 antibody, also know as "MOR03087" or "MOR3087". The terms are used interchangeable in the present disclosure.

The amino acid sequence of the MOR202 HCDR1 as defined by Kabat is:
SYYMN (SEQ ID NO: 1)

The amino acid sequence of the MOR202 HCDR2 as defined by Kabat is:
GISGDPSNTYYADSVKG (SEQ ID NO: 2)

The amino acid sequence of the MOR202 HCDR3 as defined by Kabat is:
DLPLVYTGFAY (SEQ ID NO: 3)

The amino acid sequence of the MOR202 LCDR1 as defined by Kabat is:
SGDNLRHYYVY (SEQ ID NO: 4)

The amino acid sequence of the MOR202 LCDR2 as defined by Kabat is:
GDSKRPS (SEQ ID NO: 5)

The amino acid sequence of the MOR202 LCDR3 is:
QTYTGGASL (SEQ ID NO: 6)

The amino acid sequence of the MOR202 Variable Heavy Domain is:

QVQLVESGGGLVQPGGSLRLSCAASGFTFSSYYMNWVRQAPGKGLEWVSGISGDPSNTYYADS
VKGRFTISRDNSKNTLYLQMNSLRAEDTAVYYCARDLPLVYTGFAYWGQGTLVTVSS (SEQ ID
NO: 7)

The amino acid sequence of the MOR202 Variable Light Domain is:

DIELTQPPSVSVAPGQTARISCSGDNLRHYYVYWYQQKPGQAPVLVIYGDSKRPSGIPERFSGSN
SGNTATLTISGTQAEDEADYYCQTYTGGASLVFGGGTKLTVLGQ (SEQ ID NO: 8)

The DNA sequence encoding the MOR202 Variable Heavy Domain is:

CAGGTGCAATTGGTGGAAAGCGGCGGCGGCCTGGTGCAACCGGGCGGCAGCCTGCGTCTGA
GCTGCGCGGCCTCCGGATTTACCTTTTCTTCTTATTATATGAATTGGGTGCGCCAAGCCCCTG
GGAAGGGTCTCGAGTGGGTGAGCGGTATCTCTGGTGATCCTAGCAATACCTATTATGCGGATA
GCGTGAAAGGCCGTTTTACCATTTCACGTGATAATTCGAAAAACACCCTGTATCTGCAAATGAA
CAGCCTGCGTGCGGAAGATACGGCCGTGTATTATTGCGCGCGTGATCTTCCTCTTGTTTATAC
TGGTTTTGCTTATTGGGGCCAAGGCACCCTGGTGACGGTTAGCTCA (SEQ ID NO: 10).

The DNA sequence encoding the MOR202 Variable Light Domain is:

GATATCGAACTGACCCAGCCGCCTTCAGTGAGCGTTGCACCAGGTCAGACCGCGCGTATCTC
GTGTAGCGGCGATAATCTTCGTCATTATTATGTTTATTGGTACCAGCAGAAACCCGGGCAGGC
GCCAGTTCTTGTGATTTATGGTGATTCTAAGCGTCCCTCAGGCATCCCGGAACGCTTTAGCGG
ATCCAACAGCGGCAACACCGCGACCCTGACCATTAGCGGCACTCAGGCGGAAGACGAAGCG
GATTATTATTGCCAGACTTATACTGGTGGTGCTTCTCTTGTGTTTGGCGGCGGCACGAAGTTAA
CCGTTCTTGGCCAG (SEQ ID NO: 11).

MOR202 has an IgG1 Fc region.

The term "hematological cancer" as used herein refers to a cancer of the blood, and includes leukemia, lymphoma and myeloma among others. "Leukemia" refers to a cancer of the blood in which too many white blood cells that are ineffective in fighting infection are made, thus crowding out the other parts that make up the blood, such as platelets and red blood cells. It is understood that cases of leukemia are classified as acute or chronic. Certain forms of leukemia include, by way of nonlimiting examples, acute lymphocytic leukemia (ALL); acute myeloid leukemia (AML); chronic lymphocytic leukemia (CLL); chronic myelogenous leukemia (CML); Myeloproliferative disorder/neoplasm (MPDS); and myelodysplasia syndrome. "Lymphoma" may refer to a Hodgkin's lymphoma, both indolent and aggressive non-Hodgkin's lymphoma, Burkitt's lymphoma, and follicular lymphoma (small cell and large cell), among others. Myeloma may refer to multiple myeloma (MM), giant cell myeloma, heavy-chain myeloma, and light chain or Bence-Jones myeloma.

The term "solid tumor" or "solid cancer" as used herein refers to tumors that usually do not contain cysts or liquid areas. Solid tumors as used herein include sarcomas and carcinomas, such as e.g. breast tumors, ovarian tumors, gastric tumors, lung tumors, pancreatic tumors, prostate tumors, melanoma tumors, colorectal tumors, lung tumors, head and neck tumors, bladder tumors, esophageal tumors, liver tumors, thyroid tumors and kidney tumors.

In certain embodiments, the present disclosure provides a pharmaceutical compositon for use in a method of treating a disease or disorder in a subject comprising administering to said subject an effective amount of the formulation of the present disclosure. In certain embodiments, said disease or disorder is cancer or an inflammatory disorder. In certain embodiments, said cancer is a hematological cancer or a solid cancer. In certain embodiments, said cancer is a hematological cancer is selected from leukemia, lymphoma and myeloma. In certain embodiments, said cancer is multiple myeloma. In certain embodiments, said cancer is a solid cancer selected from sarcomas and carcinomas, such as e.g. breast tumors, ovarian tumors, gastric tumors, lung tumors, pancreatic tumors, prostate tumors, melanoma tumors, colorectal tumors, lung tumors, head and neck tumors, bladder tumors, esophageal tumors, liver tumors, thyroid tumors and kidney tumors.

In certain embodiments, said inflammatory disorder is selected from inflammatory bowel disease, rheumatoid arthritis, psoriasis, amyloidosis, systemic lupus erythematosus (SLE), atopic dermatitis, Wegener's granulomatosis and psoriatic arthritis.

In certain embodiments, the present disclosure provides the use of a pharmaceutical formulation of the present disclosure for the preparation of a medicament. In certain embodiments, said use is the use for the preparation of a medicament for the treatment of a disease or a disorder. In certain embodiments, said disease or disorder is cancer or an inflammatory disorder. In certain embodiments, said cancer is a hematological cancer or a solid cancer. In certain embodiments, said cancer is a hematological cancer is selected from leukemia, lymphoma and myeloma. In certain embodiments, said cancer is multiple myeloma. In certain embodiments, said cancer is a solid cancer selected from sarcomas and carcinomas, such as e.g. breast tumors, ovarian tumors, gastric tumors lung tumors, pancreatic tumors, prostate tumors, melanoma tumors, colorectal tumors, lung tumors, head and neck tumors, bladder tumors, esophageal tumors, liver tumors, thyroid tumors and kidney tumors. In certain embodiments, said inflammatory disorder is selected from inflammatory bowel disease, rheumatoid arthritis, psoriasis, amyloidosis, systemic lupus erythematosus (SLE), atopic dermatitis, Wegener's granulomatosis and psoriatic arthritis.

The formulations according to the invention are as defined in the claims. Any formulations described below as part of the broader disclosure that do not fall within the claim scope, are not part of the claimed invention and are instead provided for context and understanding.

In one embodiment, the present disclosure provides a pharmaceutical formulation comprising an antigen binding protein, a surfactant and a buffer. In another embodiment, the present disclosure provides a pharmaceutical formulation comprising an anti-CD38 antibody, a surfactant and a buffer. In another embodiment, the present disclosure provides a pharmaceutical formulation comprising an anti-CD38 antibody, a surfactant and a buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a surfactant and a buffer, wherein, said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a surfactant and a buffer, wherein said anti-CD38 antibody is MOR202.

In one embodiment, the present disclosure provides a pharmaceutical formulation comprising an anti-CD38 antibody, a surfactant and a buffer, wherein said anti-CD38 antibody is at a concentration of at least 55 mg/ml. In one embodiment, the present disclosure provides a pharmaceutical formulation comprising an anti-CD38 antibody, a surfactant and a buffer, wherein said anti-CD38 antibody is at a concentration of 55-75 mg/ml. In one embodiment, the present disclosure provides a pharmaceutical formulation comprising an anti-CD38 antibody, a surfactant and a buffer, wherein said anti-CD38 antibody is at a concentration of 62.5-67.5 mg/ml. In one embodiment, the present disclosure provides a pharmaceutical formulation comprising an anti-CD38 antibody, a surfactant and a buffer, wherein said anti-CD38 antibody is at a concentration of about 65 mg/ml. In one embodiment, the present disclosure provides a pharmaceutical formulation comprising an anti-CD38 antibody, a surfactant and a buffer, wherein said anti-CD38 antibody is at a concentration of 65 mg/ml.

In another embodiment, the present disclosure provides a pharmaceutical formulation comprising an antigen binding protein, a surfactant and a histidine buffer. In another embodiment, the present disclosure provides a pharmaceutical formulation comprising an antigen binding protein, a surfactant and a histidine buffer of a pH between 5.5 and 6.5. In another embodiment, the present disclosure provides a pharmaceutical formulation comprising an antigen binding protein, a surfactant and a histidine buffer of a pH between 5.9 and 6.1. In another embodiment, the present disclosure provides a pharmaceutical formulation comprising an antigen binding protein, a surfactant and a histidine buffer of about 6.0. In another embodiment, the present disclosure provides a pharmaceutical formulation comprising an antigen binding protein, a surfactant and a histidine buffer of 6.0.

In another embodiment, the present disclosure provides a pharmaceutical formulation comprising an antigen binding protein, a surfactant and a histidine buffer at a concentration of 5 mM to 15 mM and of a pH between 5.9 and 6.1. In another embodiment, the present disclosure provides a pharmaceutical formulation comprising an antigen binding protein, a surfactant and a histidine buffer at a concentration of 8 mM to 12 mM and of a pH between 5.9 and 6.1. In another embodiment, the present disclosure provides a pharmaceutical formulation comprising an antigen binding protein, a surfactant and a histidine buffer at a concentration of about 10 mM and of a pH between 5.9 and 6.1. In another embodiment, the present disclosure provides a pharmaceutical formulation comprising an antigen binding protein, a surfactant and a histidine buffer at a concentration of 10 mM and of a pH between 5.9 and 6.1.

In another embodiment, the present disclosure provides a pharmaceutical formulation comprising an antigen binding protein, a surfactant and a histidine buffer at a concentration of 5 mM to 15 mM and of a pH of about 6.0. In another embodiment, the present disclosure provides a pharmaceutical formulation comprising an antigen binding protein, a surfactant and a histidine buffer at a concentration of 8 mM to 12 mM and of a pH of about 6.0. In another embodiment, the present disclosure provides a pharmaceutical formulation comprising an antigen binding protein, a surfactant and a histidine buffer at a concentration of about 10 mM and of a pH of about 6.0. In another embodiment, the present disclosure provides a pharmaceutical formulation comprising an antigen binding protein, a surfactant and a histidine buffer at a concentration of 10 mM and of a pH of about 6.0.

In another embodiment, the present disclosure provides a pharmaceutical formulation comprising an antigen binding protein, a surfactant and a histidine buffer at a concentration of 5 mM to 15 mM and of a pH of 6.0. In another embodiment, the present disclosure provides a pharmaceutical formulation comprising an antigen binding protein, a surfactant and a histidine buffer at a concentration of 8 mM to 12 mM and of a pH of 6.0. In another embodiment, the present disclosure provides a pharmaceutical formulation comprising an antigen binding protein, a surfactant and a histidine buffer at a concentration of about 10 mM and of a pH of 6.0. In another embodiment, the present disclosure provides a pharmaceutical formulation comprising an antigen binding protein, a surfactant and a histidine buffer at a concentration of 10 mM and of a pH of 6.0.

In another embodiment, the present disclosure provides a pharmaceutical formulation comprising an antigen binding protein, a non-ionic surfactant and a buffer. In other embodiment, the present disclosure provides a pharmaceutical formulation comprising an antigen binding protein, a polysorbate and a buffer. In other embodiment, the present disclosure provides a pharmaceutical formulation comprising an antigen binding protein, polysorbate 20 and a buffer. In other embodiment, the present disclosure provides a pharmaceutical formulation comprising an antigen binding protein, polysorbate 80 and a buffer. In other embodiment, the present disclosure provides a pharmaceutical formulation comprising an antigen binding protein, polysorbate 20 and a buffer, wherein said polysorbate 20 is at a concentration of 0.05% (w/w) to 0.2% (w/w). In other embodiment, the present disclosure provides a pharmaceutical formulation comprising an antigen binding protein, polysorbate 20 and a buffer, wherein said polysorbate 20 is at a concentration of about 0.1% (w/w). In other embodiment, the present disclosure provides a pharmaceutical formulation comprising an antigen binding protein, polysorbate 20 and a buffer, wherein said polysorbate 20 is at a concentration of 0.1% (w/w).

In another embodiment, the present disclosure provides a pharmaceutical formulation comprising an anti-CD38 antibody, a surfactant and a histidine buffer. In another embodiment, the present disclosure provides a pharmaceutical formulation comprising an anti-CD38 antibody, a surfactant and a histidine buffer of a pH between 5.5 and 6.5. In another embodiment, the present disclosure provides a pharmaceutical formulation comprising an anti-CD38 antibody, a surfactant and a histidine buffer of a pH between 5.9 and 6.1. In another embodiment, the present disclosure provides a pharmaceutical formulation comprising an anti-CD38 antibody, a surfactant and a histidine buffer of a pH of about 6.0. In another embodiment, the present disclosure provides a pharmaceutical formulation comprising an anti-CD38 antibody, a surfactant and a histidine buffer of a pH of 6.0.

In another embodiment, the present disclosure provides a pharmaceutical formulation comprising an anti-CD38 antibody, a surfactant and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a surfactant and a histidine buffer of a pH between 5.5 and 6.5, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a surfactant and a histidine buffer of a pH between 5.9 and 6.1, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a surfactant and a histidine buffer of a pH of about 6.0, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a surfactant and a histidine buffer of a pH of 6.0, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6.

In another embodiment, the present disclosure provides a pharmaceutical formulation comprising an anti-CD38 antibody, a surfactant and a histidine buffer, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a surfactant and a histidine buffer of a pH between 5.5 and 6.5, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a surfactant and a histidine buffer of a pH between 5.9 and 6.1, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a surfactant and a histidine buffer of a pH of about 6.0, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a surfactant and a histidine buffer of a pH of 6.0, wherein said anti-CD38 antibody has a variable heavy chain of of SEQ ID NO.: 7 and a variable light chain of SEQ ID NO.: 8.

In another embodiment, the present disclosure provides a pharmaceutical formulation comprising an anti-CD38 antibody, a surfactant and a histidine buffer, wherein said anti-CD38 antibody is MOR202. In another embodiment, the present disclosure provides a pharmaceutical formulation comprising an anti-CD38 antibody, a surfactant and a histidine buffer of a pH between 5.5 and 6.5, wherein said anti-CD38 antibody is MOR202. In another embodiment, the present disclosure provides a pharmaceutical formulation comprising an anti-CD38 antibody, a surfactant and a histidine buffer of a pH between 5.9 and 6.1, wherein said anti-CD38 antibody is MOR202. In another embodiment, the present disclosure provides a pharmaceutical formulation comprising an anti-CD38 antibody, a surfactant and a histidine buffer of a pH of about 6.0, wherein said anti-CD38 antibody is MOR202. In another embodiment, the present disclosure provides a pharmaceutical formulation comprising an anti-CD38 antibody, a surfactant and a histidine buffer of a pH of 6.0, wherein said anti-CD38 antibody is MOR202.

In another embodiment, the present disclosure provides a pharmaceutical formulation comprising an anti-CD38 antibody, a non-ionic surfactant and a histidine buffer. In another embodiment, the present disclosure provides a pharmaceutical formulation comprising an anti-CD38 antibody, a non-ionic surfactant and a histidine buffer of a pH between 5.5 and 6.5. In another embodiment, the present disclosure provides a pharmaceutical formulation comprising an anti-CD38 antibody, a non-ionic surfactant and a histidine buffer of a pH between 5.9 and 6.1. In another embodiment, the present disclosure provides a pharmaceutical formulation comprising an anti-CD38 antibody, a non-ionic surfactant and a histidine buffer of a pH of about 6.0. In another embodiment, the present disclosure provides a pharmaceutical formulation comprising an anti-CD38 antibody, a non-ionic surfactant and a histidine buffer of a pH of 6.0.

In another embodiment, the present disclosure provides a pharmaceutical formulation comprising an anti-CD38 antibody, a non-ionic surfactant and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a non-ionic surfactant and a histidine buffer of a pH between 5.5 and 6.5, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a non-ionic surfactant and a histidine buffer of a pH between 5.9 and 6.1, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a non-ionic surfactant and a histidine buffer of a pH of about 6.0, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a non-ionic surfactant and a histidine buffer of a pH of 6.0, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6.

In another embodiment, the present disclosure provides a pharmaceutical formulation comprising an anti-CD38 antibody, a non-ionic surfactant and a histidine buffer, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a non-ionic surfactant and a histidine buffer of a pH between 5.5 and 6.5, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a non-ionic surfactant and a histidine buffer of a pH between 5.9 and 6.1, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a non-ionic surfactant and a histidine buffer of a pH of about 6.0, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a non-ionic surfactant and a histidine buffer of a pH of 6.0, wherein said anti-CD38 antibody has a variable heavy chain of of SEQ ID NO.: 7 and a variable light chain of SEQ ID NO.: 8.

In another embodiment, the present disclosure provides a pharmaceutical formulation comprising an anti-CD38 antibody, a non-ionic surfactant and a histidine buffer, wherein said anti-CD38 antibody is MOR202. In another embodiment, the present disclosure provides a pharmaceutical formulation comprising an anti-CD38 antibody, a non-ionic surfactant and a histidine buffer of a pH between 5.5 and 6.5, wherein said anti-CD38 antibody is MOR202. In another embodiment, the present disclosure provides a pharmaceutical formulation comprising an anti-CD38 antibody, a non-ionic surfactant and a histidine buffer of a pH between 5.9 and 6.1, wherein said anti-CD38 antibody is MOR202. In another embodiment, the present disclosure provides a pharmaceutical formulation comprising an anti-CD38 antibody, a non-ionic surfactant and a histidine buffer of a pH of about 6.0, wherein said anti-CD38 antibody is MOR202. In another embodiment, the present disclosure provides a pharmaceutical formulation comprising an anti-CD38 antibody, a non-ionic surfactant and a histidine buffer of a pH of 6.0, wherein said anti-CD38 antibody is MOR202.

In another embodiment, the present disclosure provides a pharmaceutical formulation comprising an anti-CD38 antibody, a non-ionic surfactant and a histidine buffer. In another embodiment, the present disclosure provides a pharmaceutical formulation comprising an anti-CD38 antibody, a non-ionic surfactant and a histidine buffer of a pH between 5.5 and 6.5. In another embodiment, the present disclosure provides a pharmaceutical formulation comprising an anti-CD38 antibody, a non-ionic surfactant and a histidine buffer of a pH between 5.9 and 6.1. In another embodiment, the present disclosure provides a pharmaceutical formulation comprising an anti-CD38 antibody, a non-ionic surfactant and a histidine buffer of a pH of about 6.0. In another embodiment, the present disclosure provides a pharmaceutical formulation comprising an anti-CD38 antibody, a non-ionic surfactant and a histidine buffer of a pH of 6.0.

In another embodiment, the present disclosure provides a pharmaceutical formulation comprising an anti-CD38 antibody, a non-ionic surfactant and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a non-ionic surfactant and a histidine buffer of a pH between 5.5 and 6.5, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a non-ionic surfactant and a histidine buffer of a pH between 5.9 and 6.1, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a non-ionic surfactant and a histidine buffer of a pH of about 6.0, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a non-ionic surfactant and a histidine buffer of a pH of 6.0, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6.

In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a polysorbate 20 and a histidine buffer of a pH between 5.5 and 6.5, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a polysorbate 20 and a histidine buffer of a pH between 5.9 and 6.1, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a polysorbate 20 and a histidine buffer of a pH of about 6.0, wherein said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a polysorbate 20 and a histidine buffer of a pH of 6.0, wherein said anti-CD38 antibody has a variable heavy chain of of SEQ ID NO.: 7 and a variable light chain of SEQ ID NO.: 8.

In another embodiment, the present disclosure provides a pharmaceutical formulation comprising an anti-CD38 antibody, polysorbate 20 and a histidine buffer, wherein said anti-CD38 antibody is MOR202. In another embodiment, the present disclosure provides a pharmaceutical formulation comprising an anti-CD38 antibody, a polysorbate 20 and a histidine buffer of a pH between 5.5 and 6.5, wherein said anti-CD38 antibody is MOR202. In another embodiment, the present disclosure provides a pharmaceutical formulation comprising an anti-CD38 antibody, a polysorbate 20 and a histidine buffer of a pH between 5.9 and 6.1, wherein said anti-CD38 antibody is MOR202. In another embodiment, the present disclosure provides a pharmaceutical formulation comprising an anti-CD38 antibody, a polysorbate 20 and a histidine buffer of a pH of about 6.0, wherein said anti-CD38 antibody is MOR202. In another embodiment, the present disclosure provides a pharmaceutical formulation comprising an anti-CD38 antibody, a polysorbate 20 and a histidine buffer of a pH of 6.0, wherein said anti-CD38 antibody is MOR202.

In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, wherein said polysorbate 20 is at a concentration of 0.05% (w/w) to 0.2% (w/w). In another embodiment, the present disclosure provides a pharmaceutical formulation comprising an anti-CD38 antibody, a polysorbate 20 and a histidine buffer of a pH between 5.5 and 6.5, wherein said anti-CD38 antibody has a variable heavy chain of of SEQ ID NO.: 7 and a variable light chain of SEQ ID NO.: 8 and wherein said polysorbate 20 is at a concentration of 0.05% (w/w) to 0.2% (w/w). In another embodiment, the present disclosure provides a pharmaceutical formulation comprising an anti-CD38 antibody, a polysorbate 20 and a histidine buffer of a pH between 5.9 and 6.1, wherein said anti-CD38 antibody has a variable heavy chain of of SEQ ID NO.: 7 and a variable light chain of SEQ ID NO.: 8., and wherein said polysorbate 20 is at a concentration of 0.05% (w/w) to 0.2% (w/w). In another embodiment, the present disclosure provides a pharmaceutical formulation comprising an anti-CD38 antibody, a polysorbate 20 and a histidine buffer of a pH of about 6.0, wherein said anti-CD38 antibody has a variable heavy chain of of SEQ ID NO.: 7 and a variable light chain of SEQ ID NO.: 8., and wherein said polysorbate 20 is at a concentration of 0.05% (w/w) to 0.2% (w/w). In another embodiment, the present disclosure provides a pharmaceutical formulation comprising an anti-CD38 antibody, a polysorbate 20 and a histidine buffer of a pH of 6.0, wherein said anti-CD38 antibody has a variable heavy chain of of SEQ ID NO.: 7 and a variable light chain of SEQ ID NO.: 8 and wherein said polysorbate 20 is at a concentration of 0.05% (w/w) to 0.2% (w/w).

In another embodiment, the present disclosure provides a pharmaceutical formulation comprising an anti-CD38 antibody, polysorbate 20 and a histidine buffer, wherein said anti-CD38 antibody is MOR202 and wherein said polysorbate 20 is at a concentration of 0.05% (w/w) to 0.2% (w/w). In another embodiment, the present disclosure provides a pharmaceutical formulation comprising an anti-CD38 antibody, a polysorbate 20 and a histidine buffer of a pH between 5.5 and 6.5, wherein said anti-CD38 antibody is MOR202 and wherein said polysorbate 20 is at a concentration of 0.05% (w/w) to 0.2% (w/w). In another embodiment, the present disclosure provides a pharmaceutical formulation comprising an anti-CD38 antibody, a polysorbate 20 and a histidine buffer of a pH between 5.9 and 6.1, wherein said anti-CD38 antibody is MOR202 and wherein said polysorbate 20 is at a concentration of 0.05% (w/w) to 0.2% (w/w). In another embodiment, the present disclosure provides a pharmaceutical formulation comprising an anti-CD38 antibody, a polysorbate 20 and a histidine buffer of a pH of about 6.0, wherein said anti-CD38 antibody is MOR202 and wherein said polysorbate 20 is at a concentration of 0.05% (w/w) to 0.2% (w/w). In another embodiment, the present disclosure provides a pharmaceutical formulation comprising an anti-CD38 antibody, a polysorbate 20 and a histidine buffer of a pH of 6.0, wherein said anti-CD38 antibody is MOR202 and wherein said polysorbate 20 is at a concentration of 0.05% (w/w) to 0.2% (w/w).

In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, wherein said polysorbate 20 is at a concentration of about 0.1% (w/w). In another embodiment, the present disclosure provides a pharmaceutical formulation comprising an anti-CD38 antibody, a polysorbate 20 and a histidine buffer of a pH between 5.5 and 6.5, wherein said anti-CD38 antibody has a variable heavy chain of of SEQ ID NO.: 7 and a variable light chain of SEQ ID NO.: 8 and wherein said polysorbate 20 is at a concentration of about 0.1% (w/w). In another embodiment, the present disclosure provides a pharmaceutical formulation comprising an anti-CD38 antibody, a polysorbate 20 and a histidine buffer of a pH between 5.9 and 6.1, wherein said anti-CD38 antibody has a variable heavy chain of of SEQ ID NO.: 7 and a variable light chain of SEQ ID NO.: 8., and wherein said polysorbate 20 is at a concentration of about 0.1% (w/w). In another embodiment, the present disclosure provides a pharmaceutical formulation comprising an anti-CD38 antibody, a polysorbate 20 and a histidine buffer of a pH of about 6.0, wherein said anti-CD38 antibody has a variable heavy chain of of SEQ ID NO.: 7 and a variable light chain of SEQ ID NO.: 8., and wherein said polysorbate 20 is at a concentration of about 0.1% (w/w). In another embodiment, the present disclosure provides a pharmaceutical formulation comprising an anti-CD38 antibody, a polysorbate 20 and a histidine buffer of a pH of 6.0, wherein said anti-CD38 antibody has a variable heavy chain of of SEQ ID NO.: 7 and a variable light chain of SEQ ID NO.: 8, and wherein said polysorbate 20 is at a concentration of about 0.1% (w/w).

In another embodiment, the present disclosure provides a pharmaceutical formulation comprising an anti-CD38 antibody, polysorbate 20 and a histidine buffer, wherein said anti-CD38 antibody is MOR202 and wherein said polysorbate 20 is at a concentration of about 0.1% (w/w). In another embodiment, the present disclosure provides a pharmaceutical formulation comprising an anti-CD38 antibody, a polysorbate 20 and a histidine buffer of a pH between 5.5 and 6.5, wherein said anti-CD38 antibody is MOR202 and wherein said polysorbate 20 is at a concentration of about 0.1% (w/w). In another embodiment, the present disclosure provides a pharmaceutical formulation comprising an anti-CD38 antibody, a polysorbate 20 and a histidine buffer of a pH between 5.9 and 6.1, wherein said anti-CD38 antibody is MOR202 and wherein said polysorbate 20 is at a concentration of about 0.1% (w/w). In another embodiment, the present disclosure provides a pharmaceutical formulation comprising an anti-CD38 antibody, a polysorbate 20 and a histidine buffer of a pH of about 6.0, wherein said anti-CD38 antibody is MOR202 and wherein said polysorbate 20 is at a concentration of about 0.1% (w/w). In another embodiment, the present disclosure provides a pharmaceutical formulation comprising an anti-CD38 antibody, a polysorbate 20 and a histidine buffer of a pH of 6.0, wherein said anti-CD38 antibody is MOR202 and wherein said polysorbate 20 is at a concentration of about 0.1% (w/w).

In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, wherein said polysorbate 20 is at a concentration of 0.1% (w/w). In another embodiment, the present disclosure provides a pharmaceutical formulation comprising an anti-CD38 antibody, a polysorbate 20 and a histidine buffer of a pH between 5.5 and 6.5, wherein said anti-CD38 antibody has a variable heavy chain of of SEQ ID NO.: 7 and a variable light chain of SEQ ID NO.: 8 and wherein said polysorbate 20 is at a concentration of 0.1% (w/w). In another embodiment, the present disclosure provides a pharmaceutical formulation comprising an anti-CD38 antibody, a polysorbate 20 and a histidine buffer of a pH between 5.9 and 6.1, wherein said anti-CD38 antibody has a variable heavy chain of of SEQ ID NO.: 7 and a variable light chain of SEQ ID NO.: 8, and wherein said polysorbate 20 is at a concentration of 0.1% (w/w). In another embodiment, the present disclosure provides a pharmaceutical formulation comprising an anti-CD38 antibody, a polysorbate 20 and a histidine buffer of a pH of about 6.0, wherein said anti-CD38 antibody has a variable heavy chain of of SEQ ID NO.: 7 and a variable light chain of SEQ ID NO.: 8, and wherein said polysorbate 20 is at a concentration of 0.1% (w/w). In another embodiment, the present disclosure provides a pharmaceutical formulation comprising an anti-CD38 antibody, a polysorbate 20 and a histidine buffer of a pH of 6.0, wherein said anti-CD38 antibody has a variable heavy chain of of SEQ ID NO.: 7 and a variable light chain of SEQ ID NO.: 8, and wherein said polysorbate 20 is at a concentration of 0.1% (w/w).

In another embodiment, the present disclosure provides a pharmaceutical formulation comprising an anti-CD38 antibody, polysorbate 20 and a histidine buffer, wherein said anti-CD38 antibody is MOR202, wherein said polysorbate 20 is at a concentration of 0.1% (w/w). In another embodiment, the present disclosure provides a pharmaceutical formulation comprising an anti-CD38 antibody, a polysorbate 20 and a histidine buffer of a pH between 5.5 and 6.5, wherein said anti-CD38 antibody is MOR202 and wherein said polysorbate 20 is at a concentration of 0.1% (w/w). In another embodiment, the present disclosure provides a pharmaceutical formulation comprising an anti-CD38 antibody, a polysorbate 20 and a histidine buffer of a pH between 5.9 and 6.1, wherein said anti-CD38 antibody is MOR202 and wherein said polysorbate 20 is at a concentration of 0.1% (w/w). In another embodiment, the present disclosure provides a pharmaceutical formulation comprising an anti-CD38 antibody, a polysorbate 20 and a histidine buffer of a pH of about 6.0, wherein said anti-CD38 antibody is MOR202 and wherein said polysorbate 20 is at a concentration of 0.1% (w/w). In another embodiment, the present disclosure provides a pharmaceutical formulation comprising an anti-CD38 antibody, a polysorbate 20 and a histidine buffer of a pH of 6.0, wherein said anti-CD38 antibody is MOR202 and wherein said polysorbate 20 is at a concentration of 0.1% (w/w).

In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, and wherein said histidine buffer has a pH between 5.9 and 6.1. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, and wherein said histidine buffer has a pH of about 6.0. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, and wherein said histidine buffer has a pH of 6.0.

In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of about 0.1% (w/w) and wherein said histidine buffer has a pH between 5.9 and 6.1. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of about 0.1% (w/w) and wherein said histidine buffer has a pH of about 6.0. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of about 0.1% (w/w), and wherein said histidine buffer has a pH of 6.0.

In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of 0.1% (w/w) and wherein said histidine buffer has a pH between 5.9 and 6.1. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of 0.1% (w/w) and wherein said histidine buffer has a pH of about 6.0. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of 0.1% (w/w), and wherein said histidine buffer has a pH of 6.0.

In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, and wherein said histidine buffer has a pH between 5.9 and 6.1. In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, and wherein said histidine buffer has a pH of about 6.0. In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, and wherein said histidine buffer has a pH of 6.0.

In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, wherein said polysorbate 20 is at a concentration of about 0.1% (w/w) and wherein said histidine buffer has a pH between 5.9 and 6.1. In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, wherein said polysorbate 20 is at a concentration of about 0.1% (w/w) and wherein said histidine buffer has a pH of about 6.0. In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, wherein said polysorbate 20 is at a concentration of about 0.1% (w/w), and wherein said histidine buffer has a pH of 6.0.

In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, wherein said polysorbate 20 is at a concentration of 0.1% (w/w) and wherein said histidine buffer has a pH between 5.9 and 6.1. In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, wherein said polysorbate 20 is at a concentration of 0.1% (w/w) and wherein said histidine buffer has a pH of about 6.0. In another embodiment, the present disclosure provides a pharmaceutical formulation comprising an anti-CD38 antibody, polysorbate 20 and a histidine buffer, wherein said anti-CD38 antibody has a variable heavy chain of of SEQ ID NO.: 7 and a variable light chain of SEQ ID NO.: 8, wherein said polysorbate 20 is at a concentration of 0.1% (w/w), and wherein said histidine buffer has a pH of 6.0.

In another embodiment, the present disclosure provides a pharmaceutical formulation comprising an anti-CD38 antibody, polysorbate 20 and a histidine buffer, wherein said anti-CD38 antibody is MOR202 wherein said polysorbate 20 is at a concentration of about 0.1% (w/w) and wherein said histidine buffer has a pH between 5.9 and 6.1. In another embodiment, the present disclosure provides a pharmaceutical formulation comprising an anti-CD38 antibody, polysorbate 20 and a histidine buffer, wherein said anti-CD38 antibody is MOR202 wherein said polysorbate 20 is at a concentration of about 0.1% (w/w) and wherein said histidine buffer has a pH of about 6.0. In another embodiment, the present disclosure provides a pharmaceutical formulation comprising an anti-CD38 antibody, polysorbate 20 and a histidine buffer, wherein said anti-CD38 antibody is MOR202, and wherein said polysorbate 20 is at a concentration of about 0.1% (w/w), and wherein said histidine buffer has a pH of 6.0.

In another embodiment, the present disclosure provides a pharmaceutical formulation comprising an anti-CD38 antibody, polysorbate 20 and a histidine buffer, wherein said anti-CD38 antibody is MOR202, wherein said polysorbate 20 is at a concentration of 0.1% (w/w) and wherein said histidine buffer has a pH between 5.9 and 6.1. In another embodiment, the present disclosure provides a pharmaceutical formulation comprising an anti-CD38 antibody, polysorbate 20 and a histidine buffer, wherein said anti-CD38 antibody is MOR202, wherein said polysorbate 20 is at a concentration of 0.1% (w/w) and wherein said histidine buffer has a pH of about 6.0. In another embodiment, the present disclosure provides a pharmaceutical formulation comprising an anti-CD38 antibody, polysorbate 20 and a histidine buffer, wherein said anti-CD38 antibody is MOR202, wherein said polysorbate 20 is at a concentration of 0.1% (w/w), and wherein said histidine buffer has a pH of 6.0.

In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said histidine buffer has a pH between 5.9 and 6.1 and wherein said anti-CD38 antibody is at a concentration of 65 mg/ml. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said histidine buffer has a pH of about 6.0, and wherein said anti-CD38 antibody is at a concentration of 65 mg/ml. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said histidine buffer has a pH of 6.0, and wherein said anti-CD38 antibody is at a concentration of 65 mg/ml.

In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of about 0.1% (w/w), wherein said histidine buffer has a pH between 5.9 and 6.1, and wherein said anti-CD38 antibody is at a concentration of 65 mg/ml. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of about 0.1% (w/w), wherein said histidine buffer has a pH of about 6.0, and wherein said anti-CD38 antibody is at a concentration of 65 mg/ml.. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of about 0.1% (w/w), wherein said histidine buffer has a pH of 6.0, and wherein said anti-CD38 antibody is at a concentration of 65 mg/ml.

In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of 0.1% (w/w), wherein said histidine buffer has a pH between 5.9 and 6.1, and wherein said anti-CD38 antibody is at a concentration of 65 mg/ml. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of 0.1% (w/w), wherein said histidine buffer has a pH of about 6.0, and wherein said anti-CD38 antibody is at a concentration of 65 mg/ml.. In another embodiment, the present disclosure provides a pharmaceutical formulation comprising an anti-CD38 antibody, polysorbate 20 and a histidine buffer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6, wherein said polysorbate 20 is at a concentration of 0.1% (w/w), wherein said histidine buffer has a pH of 6.0, and wherein said anti-CD38 antibody is at a concentration of 65 mg/ml.

In one embodiment, the present disclosure provides a pharmaceutical formulation comprising an antigen binding protein, a surfactant, a buffer and a stabilizer. In another embodiment, the present disclosure provides a pharmaceutical formulation comprising an anti-CD38 antibody, a surfactant, a buffer and a stabilizer. In another embodiment, the present disclosure provides a pharmaceutical formulation comprising an anti-CD38 antibody, a surfactant, a buffer and a stabilizer, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a surfactant, a buffer, and a stabilizer, wherein, said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a surfactant, a buffer, and a stabilizer, wherein said anti-CD38 antibody is MOR202.

In one embodiment, the present disclosure provides a pharmaceutical formulation comprising an antigen binding protein, a surfactant, a buffer and sucrose. In another embodiment, the present disclosure provides a pharmaceutical formulation comprising an anti-CD38 antibody, a surfactant, a buffer and sucrose. In another embodiment, the present disclosure provides a pharmaceutical formulation comprising an anti-CD38 antibody, a surfactant, a buffer and sucrose, wherein said anti-CD38 antibody has a HCDR1 of SEQ ID NO.: 1, a HCDR2 of SEQ ID NO.: 2, a HCDR3 of SEQ ID NO.: 3, a LCDR1 of SEQ ID NO.: 4, a LCDR2 of SEQ ID NO.: 5 and a LCDR3 of SEQ ID NO.: 6. In another embodiment, the present disclosure provides a pharmaceutical formulation comprising an anti-CD38 antibody, a surfactant, a buffer, and sucrose, wherein, said anti-CD38 antibody has a variable heavy chain of of SEQ ID NO.: 7 and a variable light chain of SEQ ID NO.: 8. In another embodiment, the present disclosure provides a pharmaceutical formulation comprising an anti-CD38 antibody, a surfactant, a buffer, and sucrose, wherein said anti-CD38 antibody is MOR202.

In another embodiment, the present disclosure provides a pharmaceutical formulation comprising an anti-CD38 antibody, polysorbate 20, a histidine buffer and sucrose, wherein said anti-CD38 antibody is MOR202, wherein said polysorbate 20 is at a concentration of about 0.1% (w/w), wherein said histidine buffer is at a concentration of about 10 mM and has a pH between 5.9 and 6.1 and wherein said sucrose is at a concentration between 150 and 350 mM.

In another embodiment, the present disclosure provides a pharmaceutical formulation comprising an anti-CD38 antibody, polysorbate 20, a histidine buffer and sucrose, wherein said anti-CD38 antibody is MOR202, wherein said polysorbate 20 is at a concentration of about 0.1% (w/w), wherein said histidine buffer is at a concentration of about 10 mM and has a pH between 5.9 and 6.1 and wherein said sucrose is at a concentration between 175 and 300 mM.

In another embodiment, the present disclosure provides a pharmaceutical formulation comprising an anti-CD38 antibody, polysorbate 20, a histidine buffer and sucrose, wherein said anti-CD38 antibody is MOR202, wherein said polysorbate 20 is at a concentration of about 0.1% (w/w), wherein said histidine buffer is at a concentration of about 10 mM and has a pH between 5.9 and 6.1 and wherein said sucrose is at a concentration of about 260 mM.

In another embodiment, the present disclosure provides a pharmaceutical formulation comprising an anti-CD38 antibody, polysorbate 20, a histidine buffer and sucrose, wherein said anti-CD38 antibody is MOR202, wherein said polysorbate 20 is at a concentration of 0.1% (w/w), wherein said histidine buffer has a concentration of 10 mM and has a pH of about 6.0 and wherein said sucrose is at a concentration of 260 nM.

In another embodiment, the present disclosure provides a pharmaceutical formulation comprising an anti-CD38 antibody, polysorbate 20, a histidine buffer and sucrose, wherein said anti-CD38 antibody is MOR202 at a concentration of about 65 mg/mL, wherein said polysorbate 20 is at a concentration of about 0.1% (w/w), wherein said histidine buffer is at a concentration of about 10 mM and has a pH between 5.9 and 6.1 and wherein said sucrose is at a concentration of about 260 mM.

In another embodiment, the present disclosure provides a pharmaceutical formulation comprising an anti-CD38 antibody, polysorbate 20, a histidine buffer and sucrose, wherein said anti-CD38 antibody is MOR202 at a concentration of 65 mg/mL, wherein said polysorbate 20 is at a concentration of 0.1% (w/w), wherein said histidine buffer has a concentration of 10 mM and has a pH of about 6.0 and wherein said sucrose is at a concentration of 260 nM.

In certain embodiments, the present disclosure provides a lyophilized pharmaceutical formulation, which is prepared from any of the pharmaceutical formulations disclosed in the present invention. In certain embodiments, the present disclosure provides a lyophilized pharmaceutical formulation of an anti-CD38 antibody, prepared by the lyophilization of the pharmaceutical formulations of an anti-CD38 antibody described in the present disclosure.

In certain embodiments, the present disclosure provides a method for preparing a liquid formulation of an anti-CD38 antibody, said method comprising providing a lyophilized pharmaceutical formulation according to the present disclosure and reconstituting said lyophilized formulation.

In certain embodiments, the present disclosure provides a method for preparing a liquid formulation of an anti-CD38 antibody, said method comprising providing a lyophilized pharmaceutical formulation according to the present disclosure and reconstituting said lyophilized formulation, wherein said reconstitution is achieved via the addition of water or pharmaceutical acceptable reagent. In certain embodiments, the present disclosure provides a method for preparing a liquid formulation of an anti-CD38 antibody, said method comprising providing a lyophilized pharmaceutical formulation according to the present disclosure and reconstituting said lyophilized formulation, wherein said reconstitution is achieved via the addition of water.

In certain embodiments, the present disclosure provides a reconstituted pharmaceutical formulation of an anti-CD38 antibody obtained by reconstituting a lyophilized formulation of the present disclosure. In certain embodiments, said reconstitution is achieved via the addition of water or pharmaceutical acceptable reagent to a lyophilized solution comprising an anti-CD38 antibody. In certain embodiments, said reconstitution is achieved via the addition of water to a lyophilized solution comprising an anti-CD38 antibody.

In certain embodiments, the present disclosure provides a liquid formulation or a lyophilized formulation or a liquid formulation reconstituted from a lyophilized formulation.

In certain embodiments, the present disclosure provides a reconstituted pharmaceutical formulation comprising an anti-CD38 antibody, a surfactant and a histidine buffer of a pH between 5.5 and 6.5, wherein said anti-CD38 antibody is at a concentration of 55-75 mg/ml. In certain embodiments, the present disclosure provides a reconstituted pharmaceutical formulation comprising an anti-CD38 antibody, a surfactant and a histidine buffer of a pH between 5.5 and 6.5, wherein said anti-CD38 antibody is at a concentration of 62.5-67.5 mg/ml. In certain embodiments, the present disclosure provides a reconstituted pharmaceutical formulation comprising an anti-CD38 antibody, a surfactant and a histidine buffer of a pH between 5.5 and 6.5, wherein said anti-CD38 antibody is at a concentration of about 65 mg/ml. In certain embodiments, the present disclosure provides a reconstituted pharmaceutical formulation comprising an anti-CD38 antibody, a surfactant and a histidine buffer of a pH between 5.5 and 6.5, wherein said anti-CD38 antibody is at a concentration of 65 mg/ml.

In certain embodiments, the reconstituted pharmaceutical formulation also comprises a stabilizer. Suitable stabilizers include but are not limited to human serum albumin, bovine serum albumin, α-casein, globulins, α-lactalbumin, LDH, lysozyme, myoglobin, ovalbumin, and RNase A. Stabilizers also include amino acids and their metabolites, such as, glycine, alanine, arginine, betaine, leucine, lysine, glutamic acid, aspartic acid, proline, 4-hydroxyproline, sarcosine, γ-aminobutyric acid (GABA), opines (alanopine, octopine, strombine), and trimethylamine N-oxide (TMAO). Stabilizers may also include sugar alcohols and mono-, di- or polysaccharides, such as, mannitol, sorbitol, trehalose, dextrose, lactose, sucrose, and the like. Preferably, the stabilizer is sucrose.

In certain embodiments, the present disclosure provides a reconstituted pharmaceutical formulation comprising an anti-CD38 antibody, a surfactant, a histidine buffer of a pH between 5.5 and 6.5, and a stabilizer, wherein said anti-CD38 antibody is at a concentration of 55-75 mg/ml. In certain embodiments, the present disclosure provides a reconstituted pharmaceutical formulation comprising an anti-CD38 antibody, a surfactant, a histidine buffer of a pH between 5.5 and 6.5, and a stabilizer, wherein said anti-CD38 antibody is at a concentration of 62.5-67.5 mg/ml. In certain embodiments, the present disclosure provides a reconstituted pharmaceutical formulation comprising an anti-CD38 antibody, a surfactant, a histidine buffer of a pH between 5.5 and 6.5, and a stabilizer, wherein said anti-CD38 antibody is at a concentration of about 65 mg/ml. In certain embodiments, the present disclosure provides a reconstituted pharmaceutical formulation comprising an anti-CD38 antibody, a surfactant, a histidine buffer of a pH between 5.5 and 6.5, and a stabilizer, wherein said anti-CD38 antibody is at a concentration of 65 mg/ml.

In certain embodiments, the pharmaceutical formulations of the present disclosure are for the use in the treatment of a disease or disorder. In certain embodiments, the present disclosure provides a method of treating a disease or disorder in a subject comprising administering to said subject an effective amount of the pharmaceutical formulations of the present disclosure. In certain embodiments, the present disclosure provides the use of the pharmaceutical formulations of the present disclosure for the preparation of a medicament for the treatment of a disease or a disorder.

### Examples

### Example 1: Liquid formulation for an anti-CD38 antibody

A liquid formulation of the anti-CD38 specific antibody MOR202 was developed which is suitable for long term storage.

The formulation strategy consisted of a buffer and pH screening based on thermal stability, followed by excipient optimization with emphasis on protective characteristics against aggregate formation. The buffer screening was performed by differential scanning calorimetry (DSC) and resulted in two lead buffers: a histidine buffer (10 mM histidine pH 6.8) and a phosphate buffer (10 mM phosphate buffer pH 6.6). These buffers were combined with NaCl and sucrose in a full factorial design.

Samples were subjected to freeze-thaw as well as thermo-mechanical stress and were visually inspected for opalescence and particulate formation. Finally, the product was characterized in the different formulations by UV spectrometry, size exclusion chromatography (SEC) and dynamic light scattering (DLS). A weighted ranking of the analytical outcome identified phosphate or histidine buffer with 125 mM NaCl as most favourable compositions. Histidine buffer showed superior protective properties against freeze-thawing and was therefore chosen as basis for the final formulation.

Based on data from this study and experience from previous pre-formulation work the following formulation was selected (CMC-1 formulation):
10 mM L-histidine, pH 6.8
140 mM NaCl
0.02 % Tween 20
The CMC-1 formulation was used in a Phase I/lla clinical study.

### Example 2: Pre-formuation development for a lyophilized formulation

A pre-formulation project was initiated to develop a formulation for the drug product for the anti-CD38 specific antibody MOR202 for Phase III clinical trials and for market supply.

Objectives of this study included:
- Confirmation of the pH of maximum chemical any physical stability and the effect of protein concentration on stability of the molecule
- Evaluation of the effect of the shear forces (shaking) and freeze-thaw cycles on the physical and chemical stability of the molecule
- Determination of the stability of the protein after dilution into 0.9% NaCl saline (normal saline)

### Example 2.1: Concentration of the bulk drug substance

MOR202 bulk drug substance was concentrated by tangential flow filtration (Omega Centramate 30 kD 0.02 sqm, Pall) to a final concentration of 80-90 mg/ml. Trans-membrane pressure not exceeding 6 psi was used to monitor the tangential flow filtration process. The resulting concentrated solution was divided into sub-lots, loaded into Slide-A-Lyzer Dialysis Flasks and dialysed against the respective buffer (2.0 litre, at least 5x buffer exchanges) over 24 hours. 0.3% NaCl was added to the citrate buffer matrices to increase the ionic strength and stabilize the MOR202 solutions.

At the completion of buffer exchange the MOR202 concentration was measured. Subsequently the solutions were diluted with the respective buffer and were filtered using 0.22 µm syringe filters (PVDF, Millipore or PALL). Filtered formulations were then filled into 5 ml glass vials, stoppered and sealed.

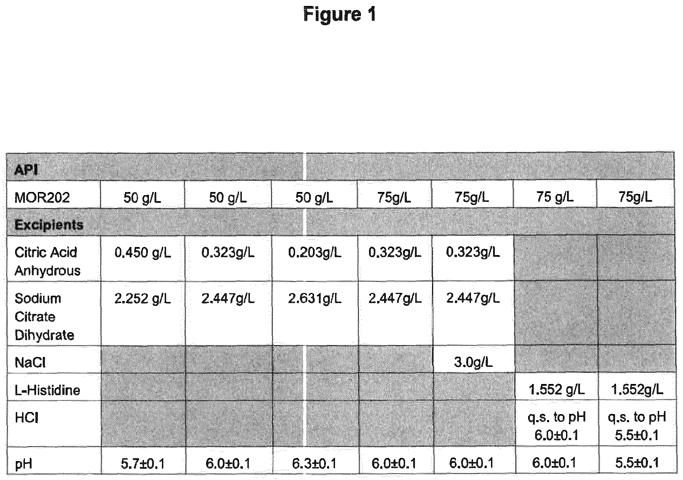

The key pre-formulations tested are summarized in Figure 1. Two different MOR202 concentrations were tested:- 50 g/l and 75 g/l. Based on pre-experiments and experience two key buffer systems were compared: histidine buffer and citrate buffer.

### Example 2.2: Stability and stress tests

The solutions prepared in Example 2.1 were subjected to various stability and stress tests. Main goal was to determine the optimum pH and to study the effect of protein concentration.

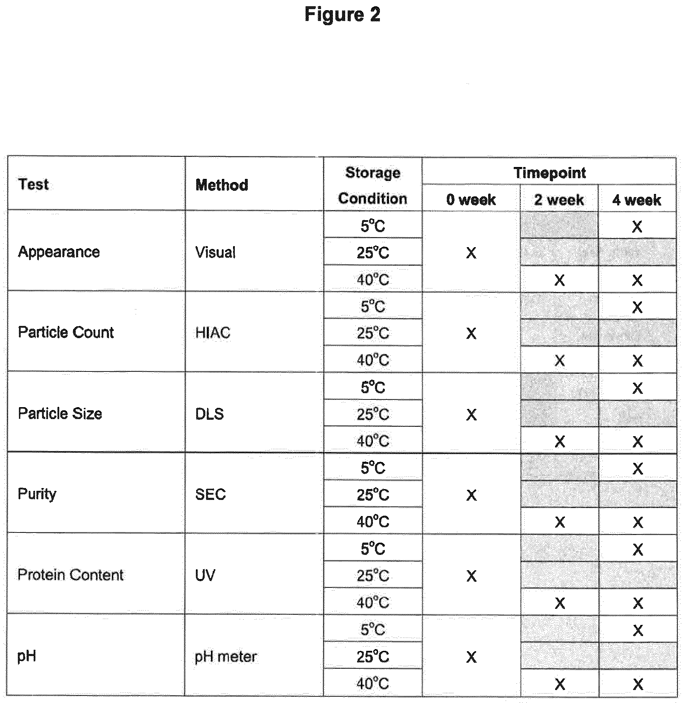

Stability was tested by storing the vials at different conditions: 5±3°C, 25±2°C / 60±5 % RH, and 40±2°C / 75±5 % RH (RH = relative humidty). Samples were investigated at certain time points. Tests perfomed as well as the test intervals are shown in Figure 2.

Shear and freeze-thaw cycles were used to investigate the concentration of the surfactant needed to effectively address any physical stability issues of the formulation (e.g., aggregation, appearance) and to protection the API during processing (e.g., mixing, pumping). From a previous study it was known that polysorbate 20 offeres protection against degradation and aggregation. The following polysorbate 20 concentrations (w/v %) were tested: 0.02%, 0.04%, and 0.1%.

For shear tests the formulation was agitated on a rotary shaker at 40-60 rpm at room temperature for 24 and 48 hours. Physical appearance (visually), purity (SEC), protein content (UV) and hydrodynamic size/soluble aggregates (DLS) were analysed.

For freeze-thaw tests the formulations were subjected to three (3) freeze-thaw cycles (-70°C). Samples were analysed as in the shear tests.

For saline dilution tests the formulations were diluted 1 : 100 with normal saline. Purity was measured via SEC. Subvisible particles were measure via HIAC.

### Example 2.3: Results of stability tests

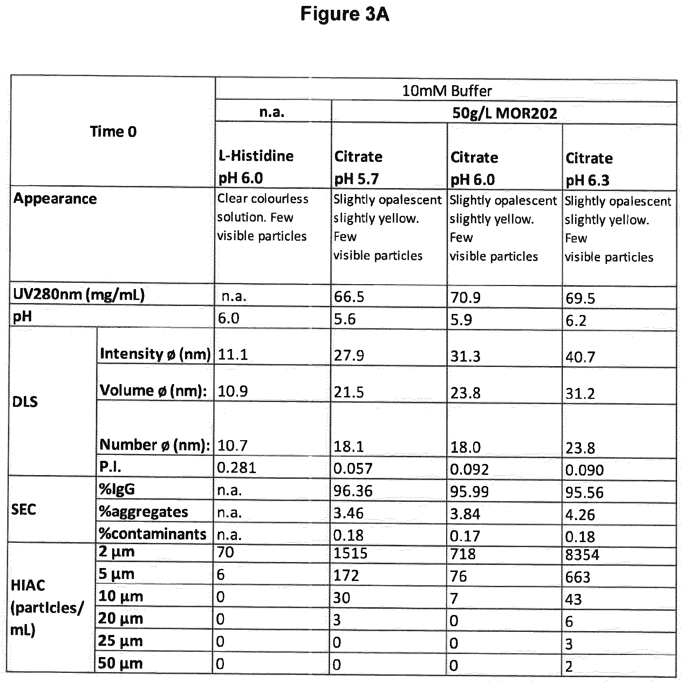

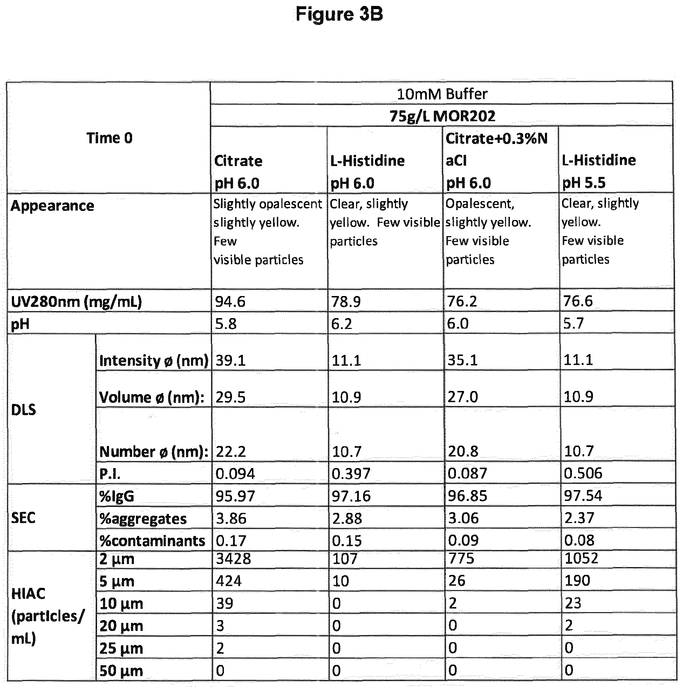

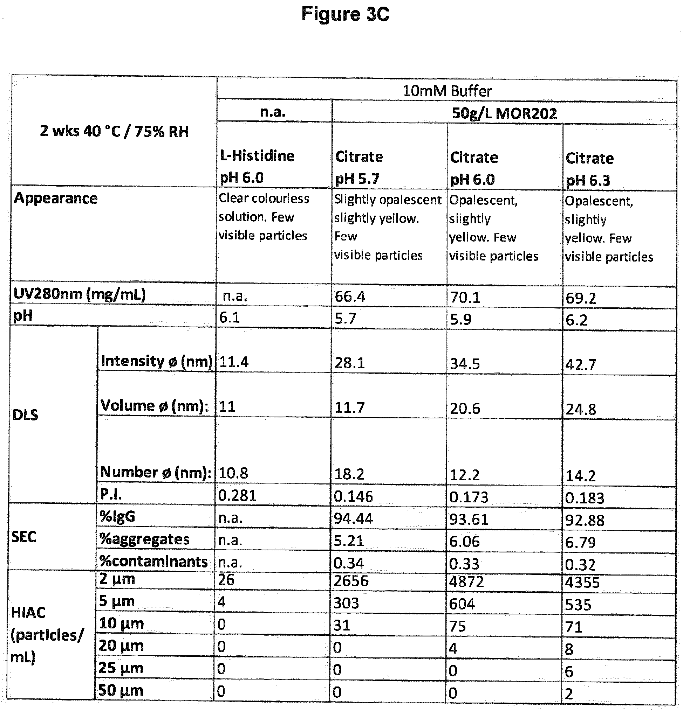

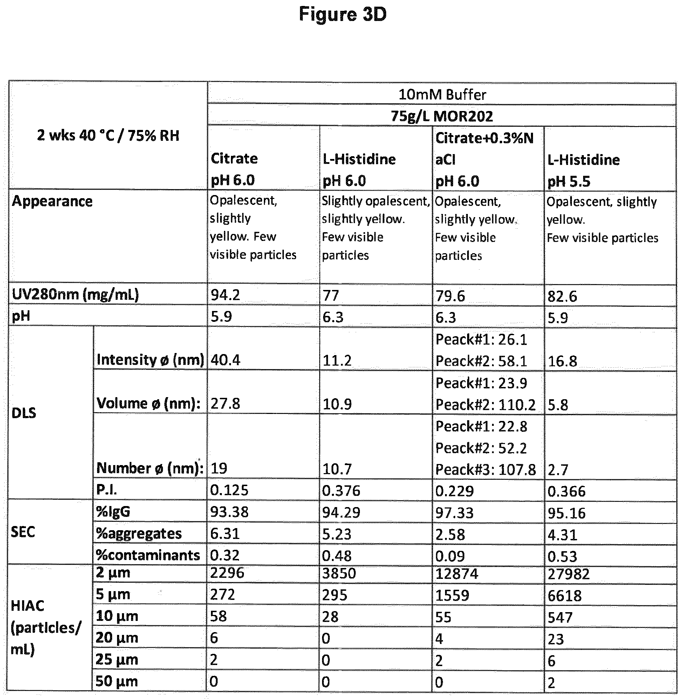

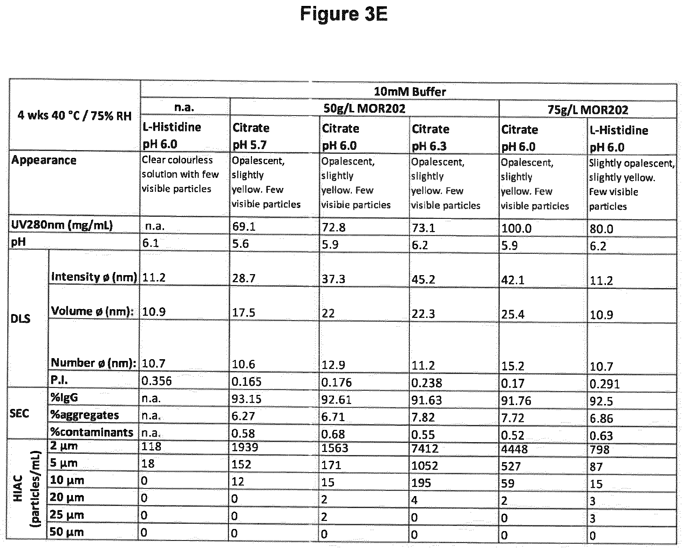

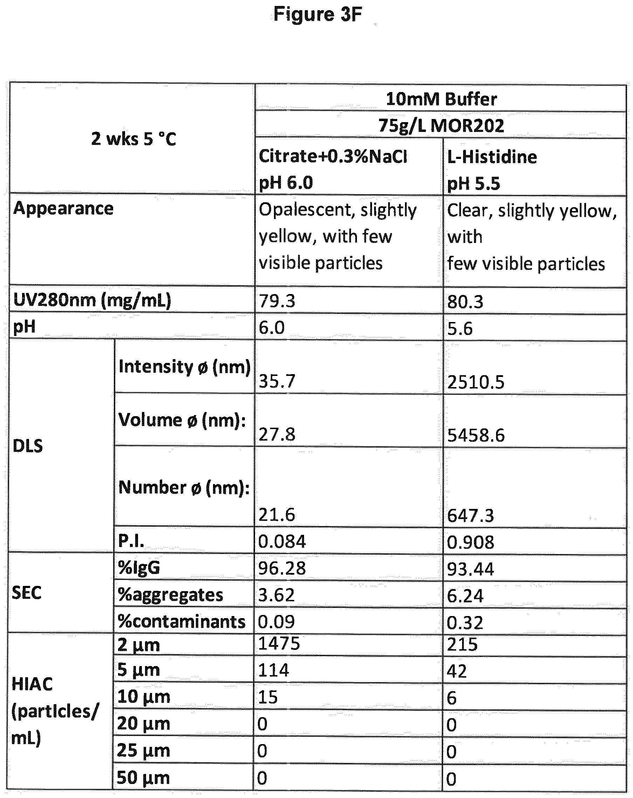

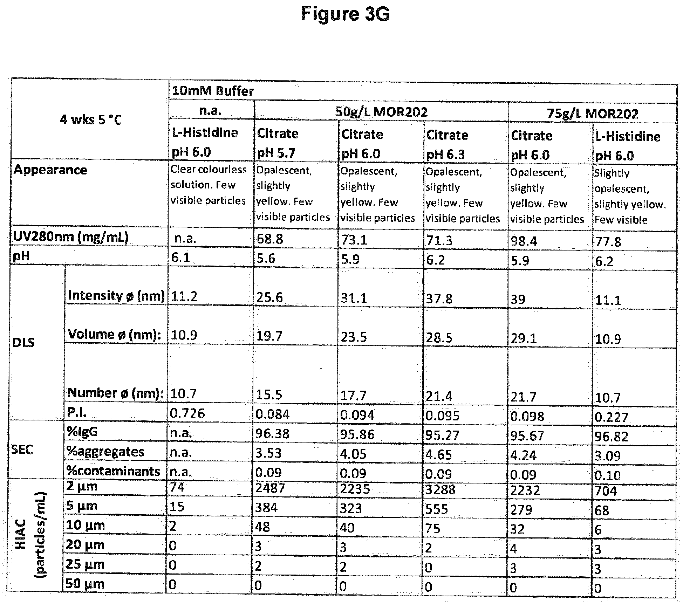

Results of the stability tests are summarized in Figures 3A-3G. The following observations were made:
- Histidine buffer is superior in stabilizing the API (MOR202).
- No significant differences in appearance and pH was observed during the stability study.
- No significant differences were detected in UV content overtime. There seems to be an increase in protein content for all formulations at the last check-point. The protein concentration increase can be ascribed to the method variability as the same increase is detected for the standard solution.
- Analysis by SEC indicates stability of all formulations at 5°C, except for the pH 5.5 L-Histidine (75 g/L) formulation that shows an increase of aggregates and contaminants after 2 weeks
- There is an increase in the number of particles per ml with increasing pH.
- Sizes assessed by DLS remain unchanged over the period of the stability study.

Taken together, the following lead buffer system was chosen as a basis for further development:

| | |
|---|---|
| MOR202 | 75 g/l |
| 10 mM buffer | |
| L-Histidine | 1.552 g/l |
| HCl | q.s. to pH 6.0 ± 0.1 |
| pH | 6.0 ± 0.1 |

Results for this lead buffer system can be described as follows:
Appearance: solutions are clear at time point zero and become slightly opalescent during the stability; colour and presence of particles remain constant
UV content: constant
pH: constant
HIAC: the number of particles during the stability remain similar for each diameter and are the lowest at time point zero
SEC: %IgG is constant at 5°C; decrease at 40°C
   %aggregates is constant at 5°C, increases at 40°C
   %contaminants is constant at 5°C, increase at 40°C
   This formulation has the lowest value of aggregates and the highest value of IgG at time point zero.
   DLS: Sizes assessed by DLS remain unchanged over the period of the stability study.

### Example 2.4: Results of shear tests, freeze/thaw tests and saline dilution tests

MOR202 solutions were tested in the lead buffer system identified in Example 2.3. The Polysorbate 20 concentration was adjusted to 0.02%, 0.04% and 0.1%.

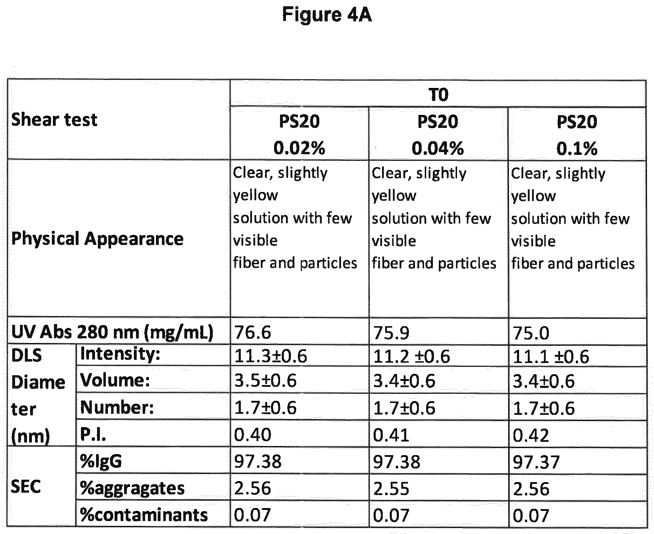

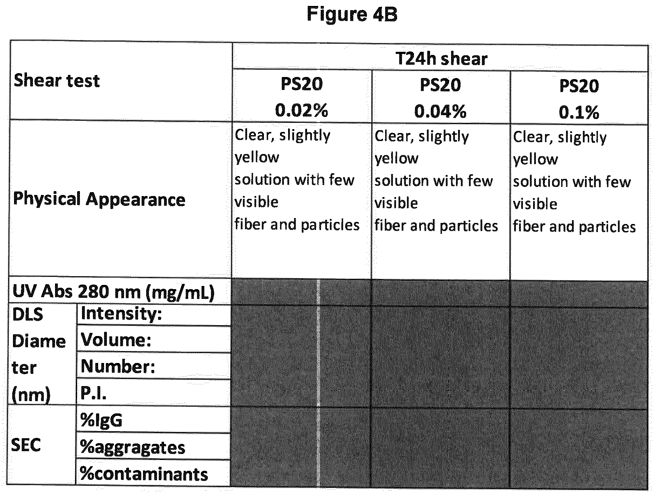

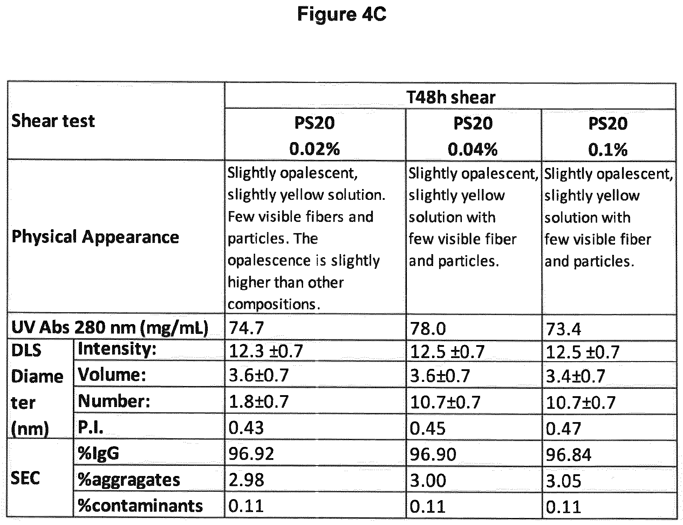

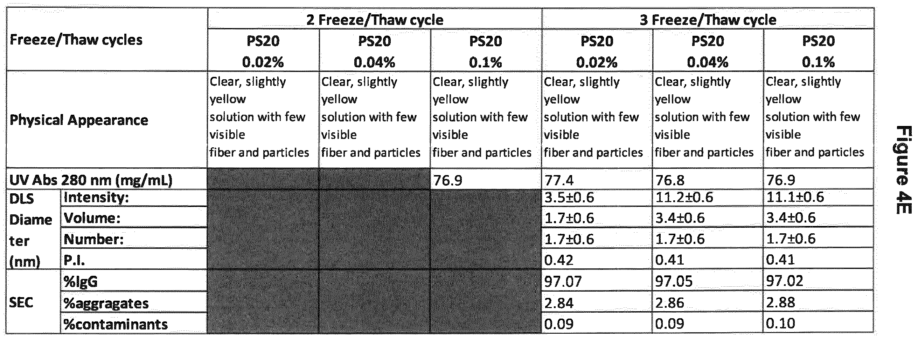

Results of the shear tests are shown in Figures 4A - 4C.

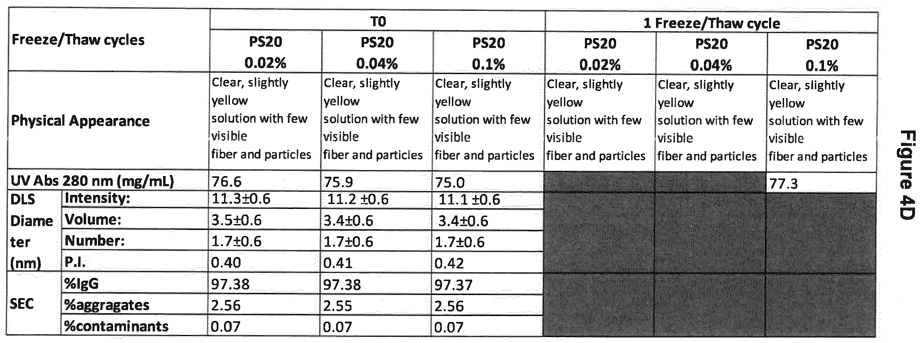

Results of the freeze/thaw tests are shown in Figures 4D - 4E.

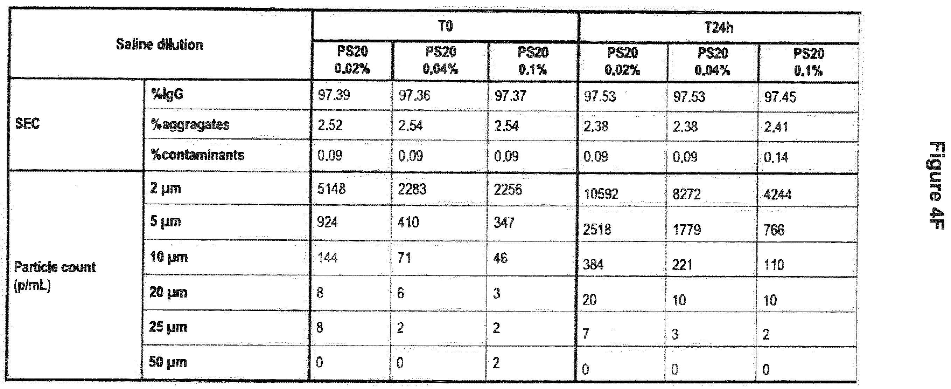

Results of the saline dilution tests are shown in Figure 4F.

### Results of the shear tests:

- The physical appearance only changes in opalescence after shearing for 48h. The formulation containing 0.02% polysorbate 20 contains the highest grade of opalescence. The shape and aspect of the fibers and particles seem not to be ascribed to the product. It is conceivable that the presence of the fibers and particles is of external origin.
- There is a slight decrease in UV content for the formulation with PS20 at concentrations of 0.04% and 0.1%.
- SEC results show that all PS20 concentrations are characterised by a small decrease of %IgG and a corresponding small increase of %aggregates.
- No major changes in sizes and polydispersity are observed when assessing the samples by DLS.

### Results of the freeze/thaw tests:

- The shape and aspect of the fibers and particles seem to not be ascribed to the product. It is conceivable that the presence of the fibers and particles is of external origin.
- The slight increase in UV content for all PS20 concentrations can be ascribed to the method variability.
- Regarding SEC results, all PS20 concentrations show a small decrease of %IgG and a corresponding small increase of % aggregates.
- Sizes assessed by DLS remain unchanged.

### Results of the saline dilution tests:

- No significant differences between time point zero and time point 24 hours in all formulations, as assessed by SEC.
- An increase in the number of particles for all diameter ranges was observed by light obscuration. The highest increase is detected in solution originally containing 0.04% Polysorbate 20, where for the 2 µm size range the number of particles increases fourfold.

### Example 2.5: Final outcome of pre-formulation development

A series of pre-formulation studies was conducted to select the optimal pH and excipients necessary to stabilize MOR202. These studies involved confirming the optimal pH between 5.5 and 6.3, studying the effect of Polysorbate 20 on freeze-thaw and agitation, as well as stability of MOR202 upon dilution in normal saline.

The molecule demonstrated good chemical and physical stability in 10 mM Histidine buffer, pH 6.0. Based on the results of these studies, 10 mM Histidine buffer pH 6.0 was considered to be the optimum matrix for the formulation of MOR202.

On the basis of the output of the shear, freeze-thaw, and dilution studies it was found that the presence of Polysorbate 20 is important and preferred.

The following composition was selected as basis for further formulation development:

| | |
|---|---|
| MOR202 | 75 g/l |
| Polysorbate 20 | 0.1% |
| 10 mM buffer | |
| L-Histidine | 1.552 g/l |
| HCl | q.s. to pH 6.0 ± 0.1 |
| pH | 6.0 ± 0.1 |

The characteristics of this formulation can be summarized as follows:
Appearance: solution becomes opalescent only after 48h of shear
UV content: shows a decrease after 48h shear and an increase after the 3^{rd} freeze/thawing cycle
SEC: small decrease of %IgG and a corresponding small increase of %aggregates after shear and freeze/thawing cycle tests. %IgG and %aggregates remain constant for saline dilution test
HIAC: increase of the number of particles for all diameters during saline dilution test. Overall number of particles significantly lower than with 0.04% PS20.
DLS: sizes asassessed by DLS remain unchanged

### Example 3: Formuation development for a lyophilized formulation of MOR202

Following pre-formulation, a new project was initiated to develop the formulation for the drug product for the anti-CD38 specific antibody MOR202.

Objectives of this study included:
- Preparation of a lyophilized MOR202 prototype formulations to be evaluated for suitability with freeze drying process;
- Analysis of the stability of the lyophilized prototype formulations;
- Selection of one lead MOR202 freeze dried formulation based on the stability data;
- Develop and optimise the freeze drying cycle based on the selected lead MOR202 formulation;
- Recommend lyophilisation cycles suitable for process transfer and scale-up at a fill/finish facility.

### Example 3.1: Thermal assessment of the prototype formulations

Based on previous knowledge and the information obtained in the pre-formulation study the following formulations were selected to be screened for freeze drying development:

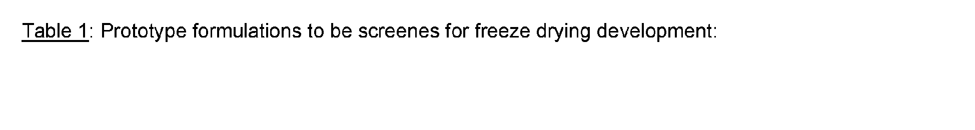

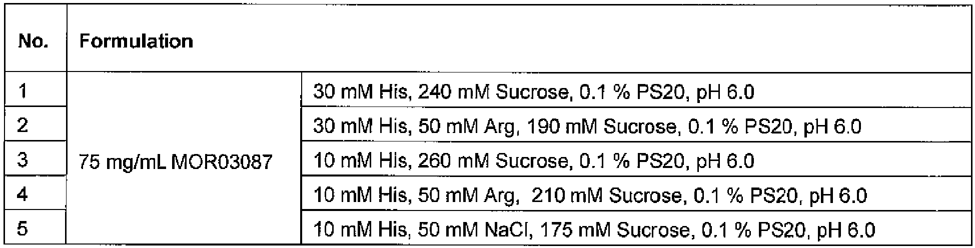

The prototype formulations were evaluated for their thermal properties, and based on the outputs of the analysis a freeze drying cycle suitable for the five prototypes was proposed.

After thermal assessment of the properties of the prototypes formulations of Table 1 the concentration of MOR202 was decreased from 75 mg/mL to 65 mg/mL. The decrease in API concentration did not effect the thermal properties of the prototype formulations.

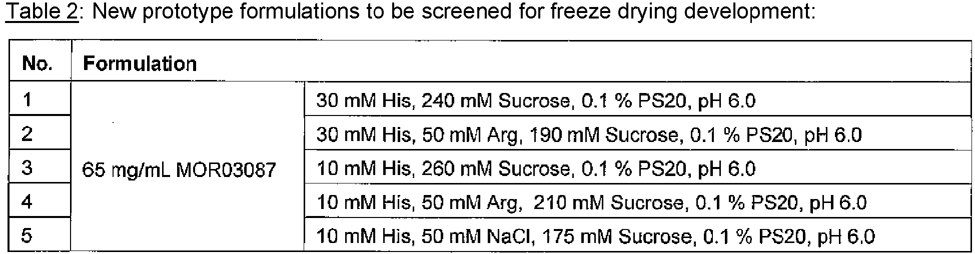

The new prototype formulations of Table 2 are refered to as F1 - F5.

An understanding of the parameters that affect the physical chemistry of freezing, freeze-drying, heat and mass transfer is key for the determination of the parameters for lyophilization cycle. Sub-ambient differential scanning calorimetry (DSC) studies were carried out with Pyris 1 Perkin Elmer system. Parameters used are shown in Table 3.

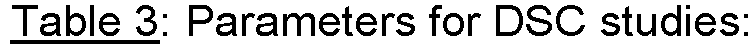

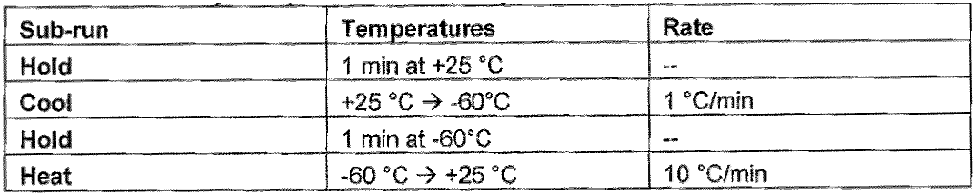

Main focus was the identification of the temperature at which crystallization occurs (eutectic point, Te) and identification of the glass transition temperature of the maximally freeze-concentrated solution (Tg'). Thermograms (freezing and heating ramps) for all prototype solutions F1-F5 were obtained. All results are summarized in Table 4:

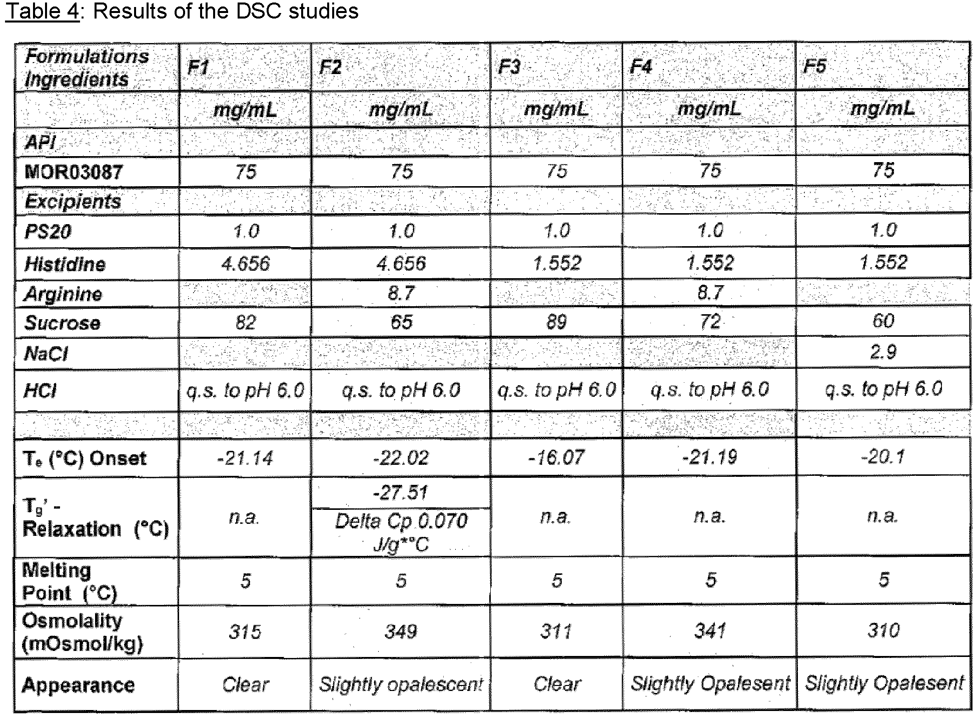

All five formulations have comparable eutectic points suggesting compatibility with one freeze drying cycle. Formulations F2, F4 and F5 appeared slightly opalescent, but no precipitate/aggregate was observed by visual inspection. Formulations F2 and F4 have an osmolality near 350 mOsmol/kg which is still in the acceptance ranges for i.v. administration. However, if the target osmolality is closer to the standard of 300 mOsmol/kg, then the sucrose content of F2 and F4 could be decreased by 12 and 10 mg, respectively. This should bring the osmolality closer to 310 mOsmol/kg without affecting the thermal properties of the formulations.

Based on these considerations the initial lyophilisation parameters were defined for the first lyo run.

### Example 3.2: Initial freeze-drying of prototype formulations

The prototype solutions F1-F5 (see Table 2) were freezed dried. The initial parameters are based on the results of the DCS studies (see Example 3.1).

The formulated bulk solutions were filtered using a 0.22 pm PVDF filter and filled into 20R vials with a filling volume of 5.00 mL + 0.3 mL overfill.

The length of the cycle was of approximately 64 hours. The cycle was monitored on real time. Primary and secondary drying were reduced from the initially proposed 50 hours to 46 hours and from 20 hours to 8 hours, respectively. The recipe is shown in Table 5.

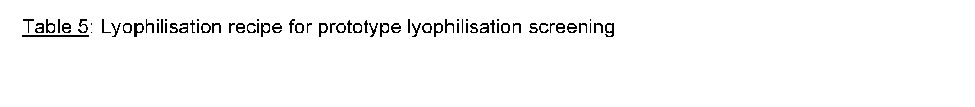

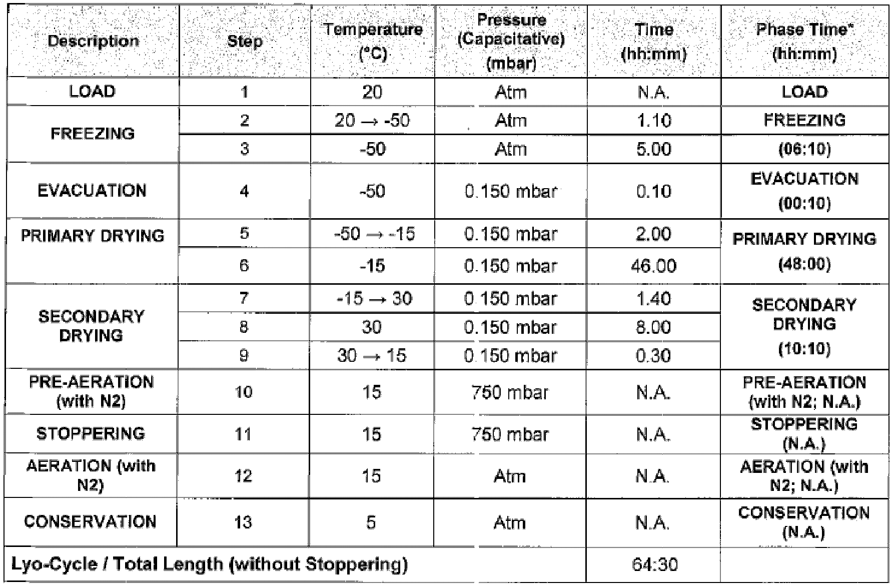

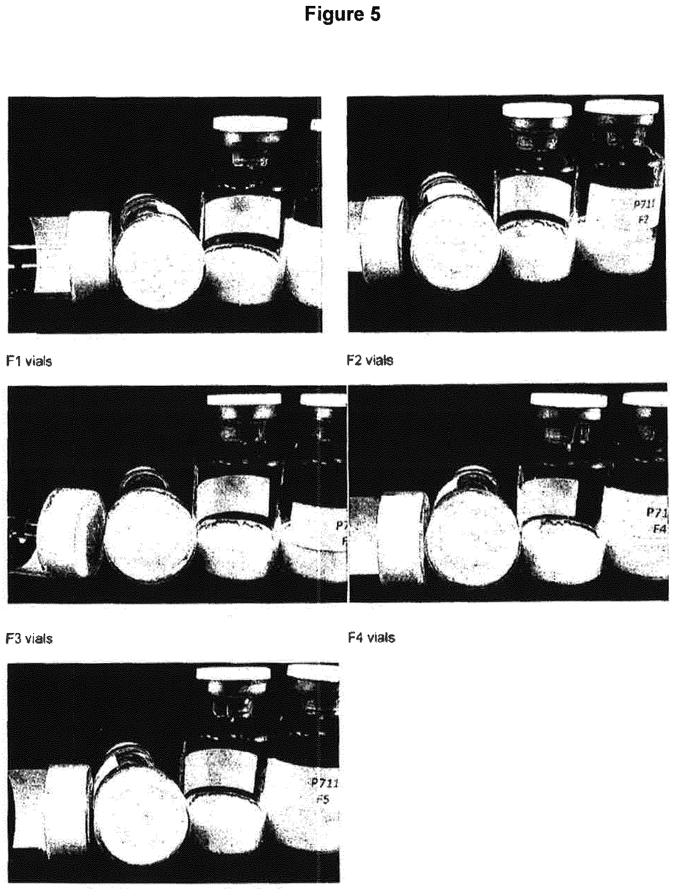

The cake of all five formulations appeared well dried. Overall, the appearance can be classified as follows (from better to less good): F3 = F5 > F2 = F4 > F1. This classification takes into account the number of cracks in the cake, the level of shrinkage, and the tendency to break. Images of vials of all formulations are shown in Figure 5.

### Example 3.3: Freezed-dried and liquid prototype formulation testing

Liquid and freeze-dried prototype formulations were analysed for the following parameters:
- Appearance (liquid and lyo)
- Moisture content (lyo only)
- pH (liquid and lyo)
- Size Exclusion Chromatography (liquid and lyo)
- Sub-Visible particles (HIAC) (liquid and lyo)
- DLS (liquid and lyo)
- Reconstitution Time (lyo)
- UV (liquid and lyo)

Stability of the formulations were also tested upon storage at various temperatures (2-8°C, 0% RH; 25°C, 60% RH; 40°C, 75% RH) and for various time points (0, 2 and 4 weeks).

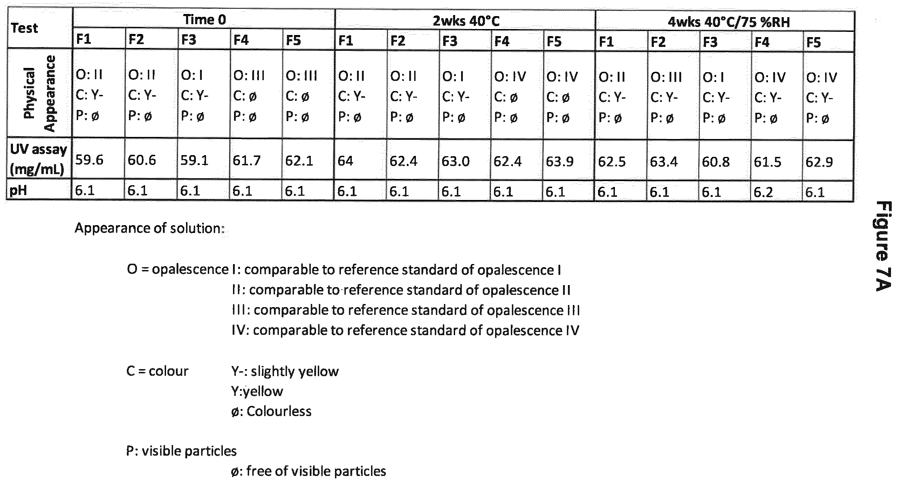

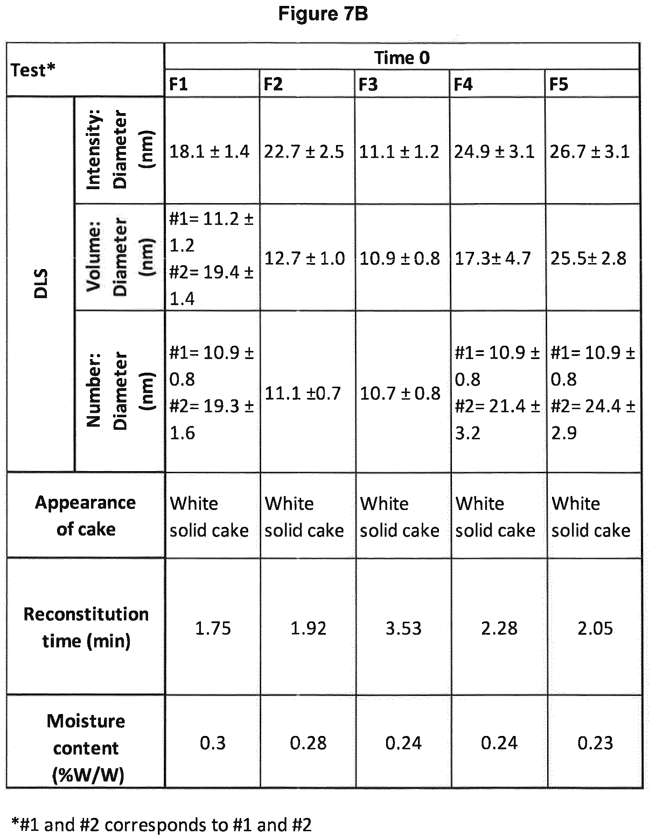

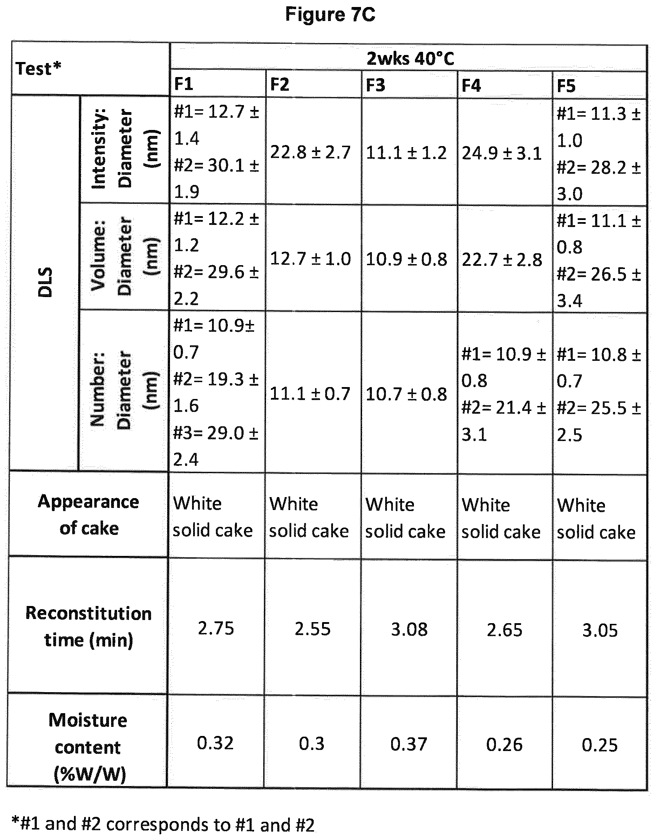

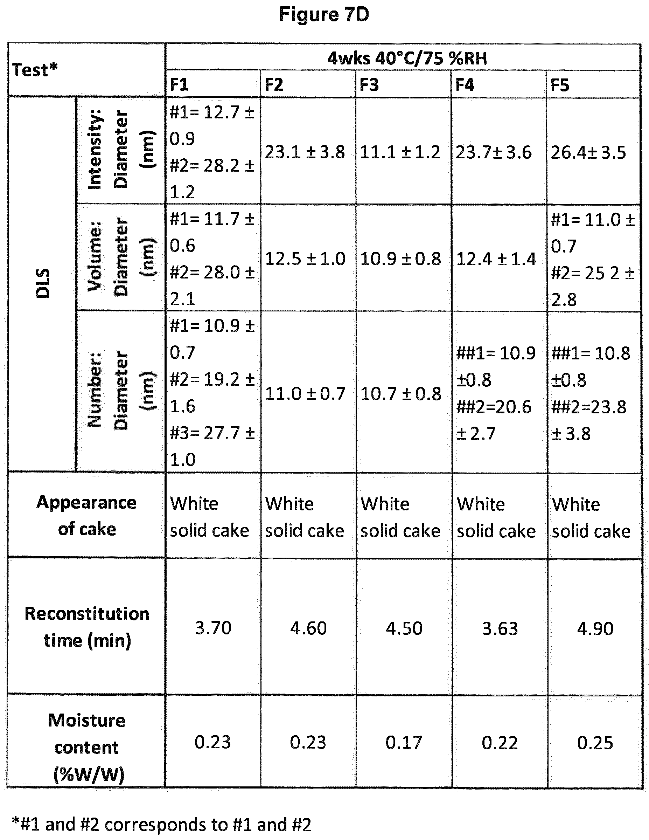

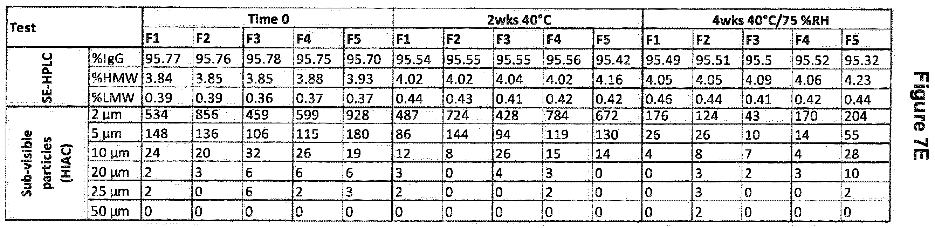

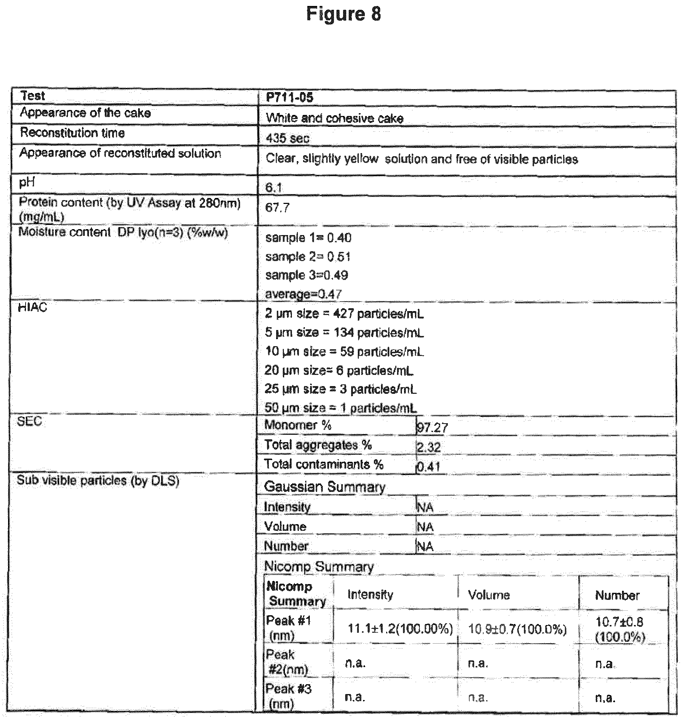

Results for the liquid formulations are shown in Figures 7A-7H. Results for the lyophilipzed formulations are shown in Figures 8A-8E.

### Example 3.4: Final results

Based on the in depth analysis of the five prototype liquid and freeze-dried formulations, formulation F3 was was selected for the development steps.

Formulation F3 showed the lowest degree of opalescence. Also, and in contrast to the other four formulations tested, no increase of sub-visible particles for the liquid formulation was seen during the stability study. Moreover, formulation F3 also showed the lowest degree of opalescense for the freeze dried formulation after reconstitution, and no increase of aggregates was observed during the stability study.

### Example 4: Optimization of lyophilization

In several subsequent steps the lyophilization process was optimized. Each subsequent process made use of the results and the experience gained during the preceeding experiments. Crucial process parameters which were evaluated in the lyophilization process are temperature, pressure and time.

### Example 4.1: First optimization

Based on the results collected in the first lyophilisation experiments (see Example 3), the process was modified. One of the main goals was to shorten the secondary drying process and to adapt the ramp rates for the different freezing/heating steps. The lyophilization recipe of the first optimization is shown in Table 6. The length of the total cycle was about 72 hours.

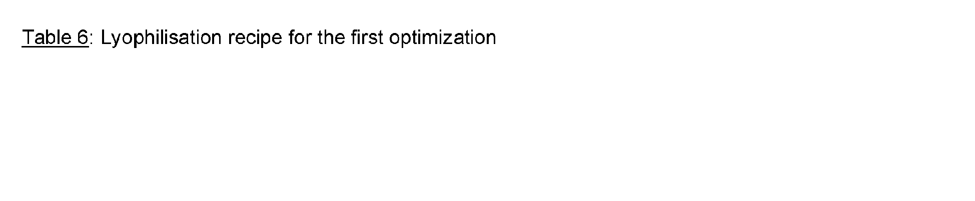

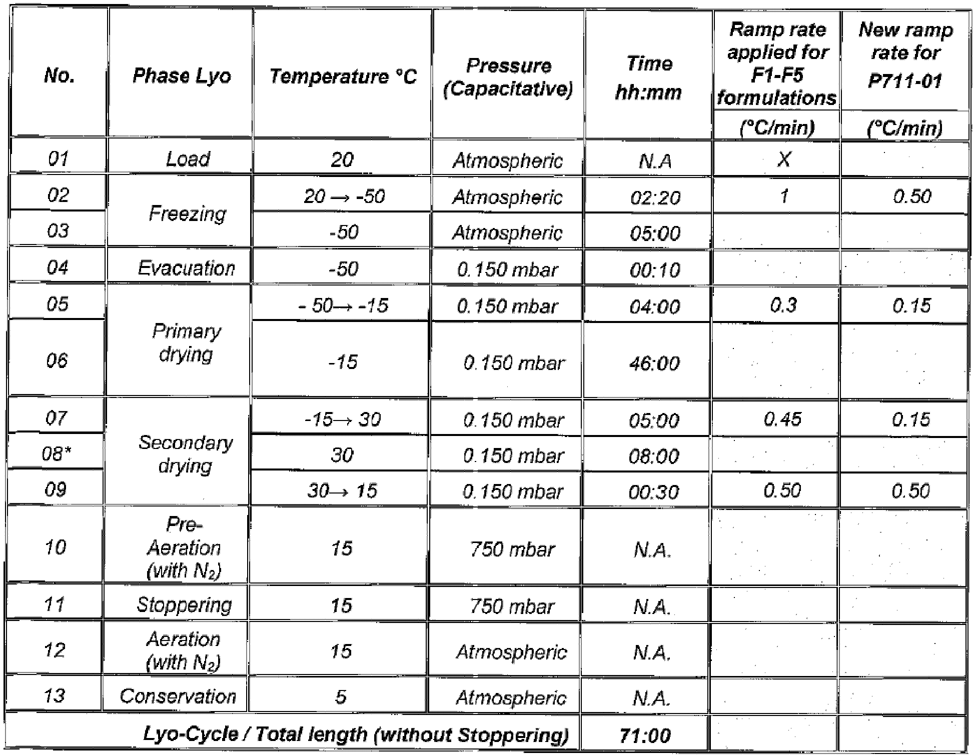

Analytical results confirmed that the lyophilized drug product showed the expected properties and did not show any anomalities.

After 5 hours of secondary drying (step 8) the residual moisture content was 0.9% w/w, so that this step had to be extended for an additional 3 hours. After 8 hours the residual moisture content was 0.3% w/w, i.e. below the target of <0.5% w/w.

It was also observed that primary drying and the follwoing ramping up of the secondary drying step can potentially be shortened. This could lead to an additional savings of about 4 hours of the entire process.

All cakes were well dried.

The appearance of the cake was classified taking into account the number of cracks in the cake, the level of shrinkage and the tendency to break.

The 24 vials tested were classified as follows:
9 vials are fine
13 vials with very slight shrinkage
2 vials with crack

Freeze-drying microscopy (FDM) was used to determine the critical temperatures of the drug product. FDM was carried out using a BTL Lyostat freeze-drying microscope (ID PDS 250).

Sample solutions of 1.5 pL were analyzed to allow the real time observation of freeze-drying of the sample. Information obtained from FDM are the collapse point (Tc) and the eutectic (Teu) point of the formulation.

Some sample display a collapse zone behaviour, i.e. the structure of the drying sample is gradually lost over a temperature range, rather than at a distinct point.

The first sign of collapse was observed at -26.8°C. With the progression of the sublimation and the increase of the temperature, also the collapse became more evident. Table summarizes the lower collapse zone temperature and recommens primary drying temperature and pressure.

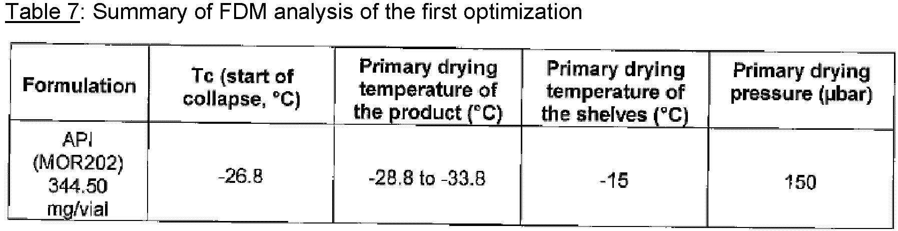

The following conclusions were drawn:
- the temperature should be ideally maintained below its respective lower collapse zone limit throughout primary drying in order to prevent product collapse
- for additional product safety and quality, a safety margin of between 2°C and 7°C, should be envisaged. This accommodates for slight variations that may occur during scale up, technology transfer and the use of different freeze-dryer
- temperature of primary drying should not be increased

### Example 4.2: Second optimization

Compared to the first optimization, in the second optimization the ramp rate for the primary and the secondary drying were increased. The primary temperature was maintained at -15°C.

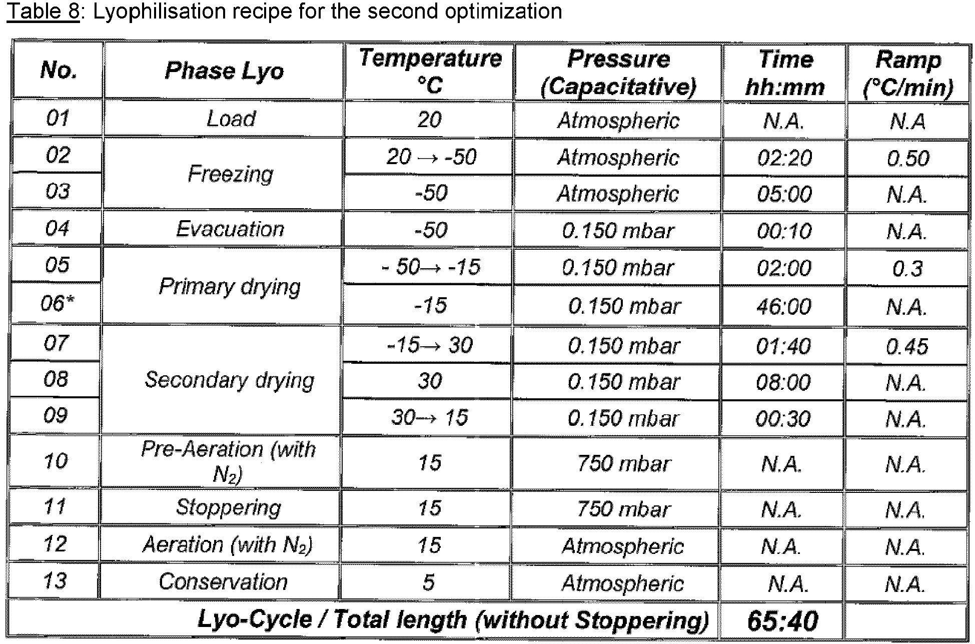

The length of the total cycle was about 66 hours, i.e. considerably shorter than in the first optimization.

Analytical results confirmed that the lyophilized drug product showed the expected properties and did not show any anomalities.

All cakes were well dried.

Again, the appearance of the cake was classified taking into account the number of cracks in the cake, the level of shrinkage and the tendency to break.

The 29 vials tested were classified as follows:
27 vials with very slight shrinkage
2 vials with very slight shrinkage and cracks on the surface

Observations made and possible changes for the next optimization round:
- the lyophilization cakes did not improve by applying different ramps and conditions; the cake appearance is probably due to excipients and the drug product
- the ramp of 1°C/min in the freezing step is not possible with all commonly used freeze driers and it is therefore recommended to work with a safety margin and use a freezing ramp of 0.5°C/min

### Example 4.3: Third optimization

The main focus of this optimization was to ensure that the quality of the drug product is not affected by the possible variations in temperature and pressure that can occur during the scale up to commercial scale. In order to test worst case conditions, temperature variations of ±3°C and pressure variations of ±30 µbars were evaluated.

Two lyophilisation experiments, HT/HP and LT/LP, were performed for the robustness study and the trials were performed at lab-scale (0.5 m² lyophilization unit) as a means of bridging between the extrapolation at laboratory scale and GMP production.

This optimization aimed to investigate high temperature and high pressure conditions.

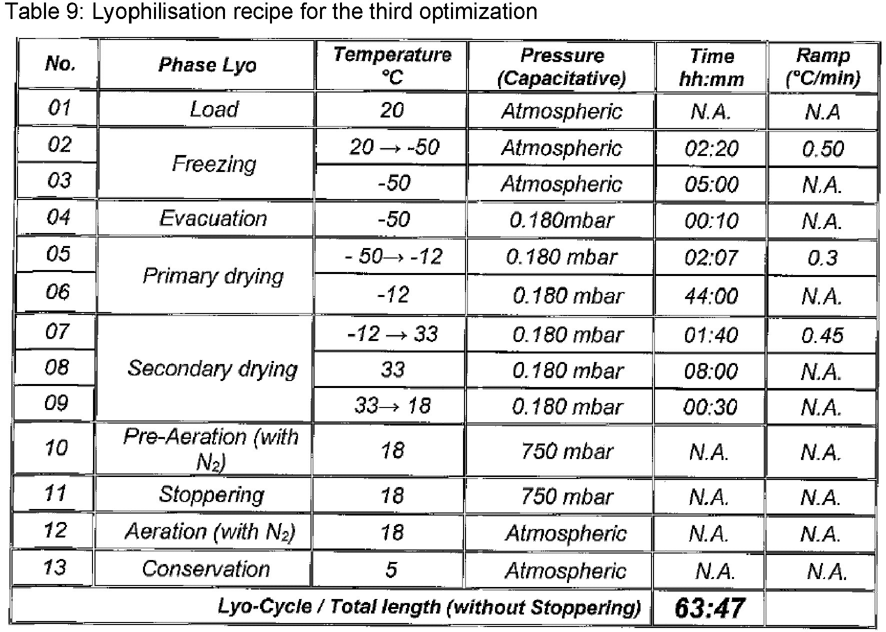

The length of the total cycle was about 64 hours, i.e. again shorter than in the last optimization.

Analytical results confirmed that the lyophilized drug product showed the expected properties and did not show any anomalities.

All cakes were well dried.

Again, the appearance of the cake was classified taking into account the number of cracks in the cake, the level of shrinkage and the tendency to break.

The 29 vials tested were classified as follows:
24 vials had a cohesive and white cake with very slight shrinkage
5 vials with very slight shrinkage and cracks on the surface

### Example 4.4: Fourth optimization

This optimization aimed to investigate low temperature and low pressure conditions.

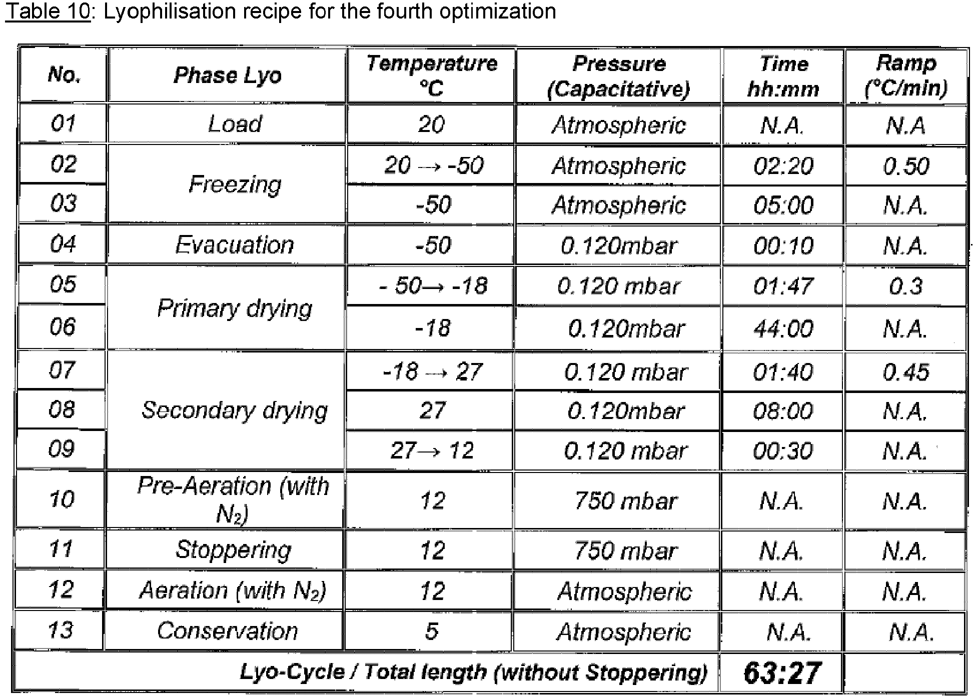

The length of the total cycle was again about 64 hours.

Analytical results confirmed that the lyophilized drug product showed the expected properties and did not show any anomalities.

All 26 cakes were well dried and cohesive. 3 vials out of the 26 showed a slight shrinkage that is not considered a defect.

The results of these runs have proven that the chosen formulation can successfully be lyophilized even with small variations of the temperature and pressure during the lyophilization process steps.

This guaratnees a robust process for large-scale routine manufacturing.

### Example 4.5: Fifth optimization

Based on all experience gather so far, a fifth optimization was performed.

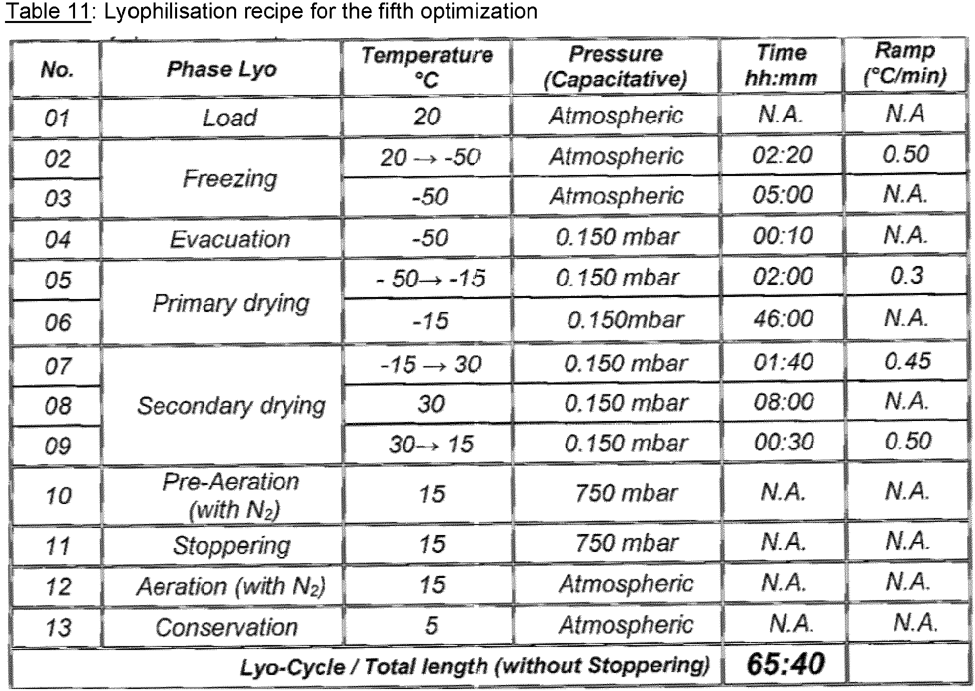

The length of the total cycle was about 66 hours.

Analytical results confirmed that the lyophilized drug product showed the expected properties and did not show any anomalities. Figure 8 shows the results of the analysis of the lyophilized drug product. Reconstitution volume was 4.8 ml. All cakes were cohesive.

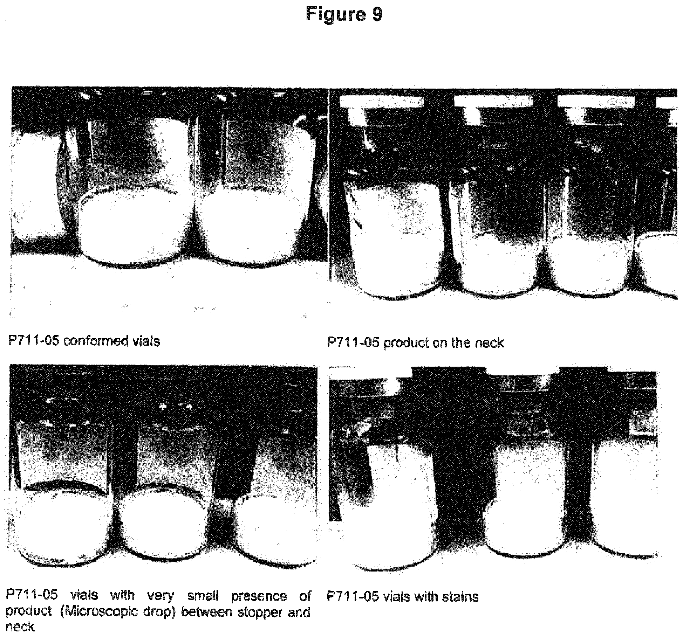

215 vials were conform. 3 out of the 215 conform vials showed cracks on the surface that is not to be considered a defect. 60 vials were rejected because of drug product between the stopper and the neck. This defect is not related to the lyophilization process but to the filling process. 44 vials either showed stains on the vials near the stopper or the presence of very small ammunts of product between the stopper and the rack. This defect was probably caused during the freeze-dryer loading. Pictures of exemplary cakes are shown in Figure 9.

The results of the fifth optimization showed that the process is robust and suited to be applied for the production of GMP material.

## Claims

1. A lyophilized pharmaceutical formulation comprising an anti-CD38 antibody, a non-ionic surfactant and a histidine buffer of a pH between 5.5 and 6.5, wherein said anti-CD38 antibody comprises a variable heavy chain of SEQ ID NO.: 7 and a variable light chain of SEQ ID NO.: 8.

2. The lyophilized pharmaceutical formulation of claim 1, wherein said non-ionic surfactant is polysorbate 20.

3. The lyophilized pharmaceutical formulation according to claim 1 or 2, wherein said histidine buffer has:
a pH of 6 to 6.5; or
a pH of 6.0.

4. The lyophilized pharmaceutical formulation according to any of claims 1 to 3, wherein the non-ionic surfactant is at a concentration of:
0.05% (w/w) to 0.2% (w/w); or
0.1% (w/w) to 0.2% (w/w).

5. The lyophilized pharmaceutical formulation according to any one of the preceding claims, further comprising sucrose.

6. A method for preparing a liquid formulation of an anti-CD38 antibody, said method comprising providing the lyophilized pharmaceutical formulation of any of the preceding claims, and reconstituting said lyophilized formulation via the addition of water.

7. A reconstituted pharmaceutical formulation of an anti-CD38 antibody obtained by the method of claim 6.

8. A reconstituted pharmaceutical formulation according to claim 7, wherein said non-ionic surfactant is at a concentration of 0.1% (w/w).

9. A reconstituted pharmaceutical formulation according to any one of claims 7 to 8, wherein said histidine buffer is at a concentration of 5 mM to 15 mM, preferably 10 mM.

10. A reconstituted pharmaceutical formulation according to any one of claims 7 to 9, wherein said sucrose is at a concentration of 150 to 350 mM, preferably 260 mM.

11. A reconstituted pharmaceutical formulation according to any one of claims 7 to 10, wherein said anti-CD38 antibody is at a concentration of 55 to 75 mg/ml, preferably 65 mg/ml.

12. A pharmaceutical formulation of any one of claims 1-5 or 7-11 for use in the treatment of a disease or disorder.

13. A pharmaceutical formulation for use according to claim 12, wherein said disease or disorder is cancer.

14. A pharmaceutical formulation for use according to claim 13, wherein said cancer is a hematological cancer is selected from leukemia, lymphoma and myeloma, such as acute lymphocytic leukemia (ALL), acute myeloid leukemia (AML), chronic lymphocytic leukemia (CLL), chronic myelogenous leukemia (CML) and multiple myeloma (MM) or a solid cancer selected from sarcoma and carcinoma, such as breast cancer, ovarian cancer, gastric cancer, lung cancer, pancreatic cancer, prostate cancer, melanoma tumors, colorectal cancer, head and neck cancer, bladder cancer, esophageal cancer, liver cancer, thyroid cancer and kidney cancer.

15. A pharmaceutical formulation for use according to claim 12, wherein said disease or disorder is an inflammatory disorder.

16. A pharmaceutical formulation for use according to claim 15, wherein said inflammatory disorder is selected from inflammatory bowel disease, rheumatoid arthritis, psoriasis, amyloidosis, systemic lupus erythematosus (SLE), atopic dermatitis, Wegener's granulomatosis, psoriatic arthritis.

## Patentansprüche

1. Lyophilisierte pharmazeutische Formulierung, umfassend einen Anti-CD38-Antikörper, ein nichtionisches Tensid und einen Histidinpuffer mit einem pH-Wert zwischen 5,5 und 6,5, wobei der Anti-CD38-Antikörper eine variable schwere Kette der SEQ ID NO.: 7 und eine variable leichte Kette der SEQ ID NO.: 8 umfasst.

2. Lyophilisierte pharmazeutische Formulierung nach Anspruch 1, wobei das nichtionische Tensid Polysorbat 20 ist.

3. Lyophilisierte pharmazeutische Formulierung nach Anspruch 1 oder 2, wobei der Histidinpuffer Folgendes aufweist:
einen pH-Wert von 6 bis 6,5; oder
einen pH-Wert von 6,0.

4. Lyophilisierte pharmazeutische Formulierung nach einem der Ansprüche 1 bis 3, wobei das nichtionische Tensid in einer Konzentration wie folgt vorliegt:
0,05 % (w/w) bis 0,2 % (w/w); oder
0,1 % (w/w) bis 0,2 % (w/w).

5. Lyophilisierte pharmazeutische Formulierung nach einem der vorhergehenden Ansprüche, ferner umfassend Saccharose.

6. Verfahren zur Herstellung einer flüssigen Formulierung eines Anti-CD38-Antikörpers, wobei das Verfahren ein Bereitstellen der lyophilisierten pharmazeutischen Formulierung nach einem der vorhergehenden Ansprüche und ein Rekonstituieren der lyophilisierten Formulierung über Zugabe von Wasser umfasst.

7. Rekonstituierte pharmazeutische Formulierung eines Anti-CD38-Antikörpers, erhalten durch das Verfahren nach Anspruch 6.

8. Rekonstituierte pharmazeutische Formulierung nach Anspruch 7, wobei das nichtionische Tensid in einer Konzentration von 0,1 % (w/w) vorliegt.

9. Rekonstituierte pharmazeutische Formulierung nach einem der Ansprüche 7 bis 8, wobei der Histidinpuffer in einer Konzentration von 5 mM bis 15 mM, vorzugsweise 10 mM, vorliegt.

10. Rekonstituierte pharmazeutische Formulierung nach einem der Ansprüche 7 bis 9, wobei die Saccharose in einer Konzentration von 150 mM bis 350 mM, vorzugsweise 260 mM, vorliegt.

11. Rekonstituierte pharmazeutische Formulierung nach einem der Ansprüche 7 bis 10, wobei der Anti-CD38-Antikörper in einer Konzentration von 55 bis 75 mg/ml, vorzugsweise 65 mg/ml, vorliegt.

12. Pharmazeutische Formulierung nach einem der Ansprüche 1 bis 5 oder 7 bis 11 zur Verwendung bei der Behandlung einer Erkrankung oder Störung.

13. Pharmazeutische Formulierung zur Verwendung nach Anspruch 12, wobei die Erkrankung oder Störung Krebs ist.

14. Pharmazeutische Formulierung zur Verwendung nach Anspruch 13, wobei der Krebs ein hämatologischer Krebs ist, ausgewählt aus Leukämie, Lymphom und Myelom, wie akute lymphatische Leukämie (ALL), akute myeloische Leukämie (AML), chronische lymphatische Leukämie (CLL), chronische myeloische Leukämie (CML) und multiples Myelom (MM), oder ein solider Krebs, ausgewählt aus Sarkom und Karzinom, wie Brustkrebs, Eierstockkrebs, Magenkrebs, Lungenkrebs, Bauchspeicheldrüsenkrebs, Prostatakrebs, Melanome, Darmkrebs, Kopf- und Halskrebs, Blasenkrebs, Speiseröhrenkrebs, Leberkrebs, Schilddrüsenkrebs und Nierenkrebs.

15. Pharmazeutische Formulierung zur Verwendung nach Anspruch 12, wobei die Erkrankung oder Störung eine entzündliche Störung ist.

16. Pharmazeutische Formulierung zur Verwendung nach Anspruch 15, wobei die entzündliche Störung ausgewählt ist aus entzündlichen Darmerkrankungen, rheumatoider Arthritis, Psoriasis, Amyloidose, systemischem Lupus erythematodes (SLE), atopischer Dermatitis, Wegener-Granulomatose, Psoriasis-Arthritis.

## Revendications

1. Formulation pharmaceutique lyophilisée comprenant un anticorps anti-CD38, un tensioactif non ionique et un tampon d'histidine présentant un pH compris entre 5,5 et 6,5, dans laquelle ledit anticorps anti-CD38 comprend une chaîne lourde variable de SEQ ID NO. : 7 et une chaîne légère variable de SEQ ID NO. : 8.

2. Formulation pharmaceutique lyophilisée selon la revendication 1, dans laquelle ledit tensioactif non ionique est le polysorbate 20.

3. Formulation pharmaceutique lyophilisée selon la revendication 1 ou 2, dans laquelle ledit tampon d'histidine présente :
un pH de 6 à 6,5 ; ou
un pH de 6,0.

4. Formulation pharmaceutique lyophilisée selon l'une quelconque des revendications 1 à 3, dans laquelle le tensioactif non ionique est présent à une concentration de :
0,05 % (p/p) à 0,2 % (p/p) ; ou
0,1 % (p/p) à 0,2 % (p/p).

5. Formulation pharmaceutique lyophilisée selon l'une quelconque des revendications précédentes, comprenant en outre du saccharose.

6. Procédé de préparation d'une formulation liquide d'un anticorps anti-CD38, ledit procédé comprenant la mise à disposition de la formulation pharmaceutique lyophilisée selon l'une des revendications précédentes et la reconstitution de ladite formulation lyophilisée par addition d'eau.

7. Formulation pharmaceutique reconstituée d'un anticorps anti-CD38 obtenue par le procédé de la revendication 6.

8. Formulation pharmaceutique reconstituée selon la revendication 7, dans laquelle ledit tensioactif non ionique est présent à une concentration de 0,1 % (p/p).

9. Formulation pharmaceutique reconstituée selon l'une quelconque des revendications 7 à 8, dans laquelle ledit tampon d'histidine est présent à une concentration de 5 mM à 15 mM, de préférence 10 mM.

10. Formulation pharmaceutique reconstituée selon l'une quelconque des revendications 7 à 9, dans laquelle ledit saccharose est présent à une concentration de 150 à 350 mM, de préférence 260 mM.

11. Formulation pharmaceutique reconstituée selon l'une quelconque des revendications 7 à 10, dans laquelle ledit anticorps anti-CD38 est présent à une concentration de 55 à 75 mg/ml, de préférence 65 mg/ml.

12. Formulation pharmaceutique selon l'une quelconque des revendications 1 à 5 ou 7 à 11 destinée à être utilisée dans le traitement d'une maladie ou d'un trouble.

13. Formulation pharmaceutique destinée à être utilisée selon la revendication 12, dans laquelle ladite maladie ou ledit trouble est un cancer.

14. Formulation pharmaceutique destinée à être utilisée selon la revendication 13, dans laquelle ledit cancer est un cancer hématologique choisi parmi la leucémie, le lymphome et le myélome, tels que la leucémie aiguë lymphoblastique (LAL), la leucémie aiguë myéloïde (LAM), la leucémie lymphoïde chronique (LLC), la leucémie myéloïde chronique (LMC) et le myélome multiple (MM), ou un cancer solide choisi parmi le sarcome et le carcinome, tels que le cancer du sein, le cancer de l'ovaire, le cancer gastrique, le cancer du poumon, le cancer du pancréas, le cancer de la prostate, les mélanomes, le cancer colorectal, le cancer de la tête et du cou, le cancer de la vessie, le cancer de l'œsophage, le cancer du foie, le cancer de la thyroïde et le cancer du rein.

15. Formulation pharmaceutique destinée à être utilisée selon la revendication 12, dans laquelle ladite maladie ou ledit trouble est un trouble inflammatoire.

16. Formulation pharmaceutique destinée à être utilisée selon la revendication 15, dans laquelle ledit trouble inflammatoire est choisi parmi les maladies inflammatoires de l'intestin, la polyarthrite rhumatoïde, le psoriasis, l'amylose, le lupus érythémateux systémique (LES), la dermatite atopique, la granulomatose de Wegener et le rhumatisme psoriasique.
